(19) Europäisches Patentamt / European Patent Office / Office européen des brevets

(11) **EP 4 183 447 B1**

(12) **EUROPEAN PATENT SPECIFICATION**

(45) Date of publication and mention
of the grant of the patent:
**10.04.2024 Bulletin 2024/15**

(21) Application number: **21208699.5**

(22) Date of filing: **17.11.2021**

(51) International Patent Classification (IPC):
**A61N 5/10** (2006.01)     **G21K 1/10** (2006.01)

(52) Cooperative Patent Classification (CPC):
**A61N 5/10; G21K 1/10;** A61N 2005/1087;
A61N 2005/1092; A61N 2005/1095

(54) **METHOD FOR DESIGNING A RIDGE FILTER IN A PBS TREATMENT SYSTEM**

VERFAHREN ZUM ENTWERFEN EINES KAMMFILTERS IN EINEM PBS-BEHANDLUNGSSYSTEM

PROCÉDÉ DE CONCEPTION D'UN FILTRE À NERVURE DANS UN SYSTÈME DE TRAITEMENT PBS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**24.05.2023 Bulletin 2023/21**

(73) Proprietor: **Ion Beam Applications**
**1348 Louvain-la-Neuve (BE)**

(72) Inventors:
• **LABARBE, Rudi**
**1348 Louvain-la-Neuve (BE)**
• **HOTOIU, Lucian**
**1348 Louvain-la-Neuve (BE)**
• **PIN, Arnaud**
**1348 Louvain-la-Neuve (BE)**

(74) Representative: **Connor, Marco Tom et al**
**Pecher & Partners**
**Rue Louis de Geer, 6**
**1348 Louvain-la-Neuve (BE)**

(56) References cited:
• **YURI SIMEONOV ET AL: "3D range-modulator for scanned particle therapy: development, Monte Carlo simulations and experimental evaluation", PHYSICS IN MEDICINE AND BIOLOGY, INSTITUTE OF PHYSICS PUBLISHING, BRISTOL GB, vol. 62, no. 17, 11 August 2017 (2017-08-11), pages 7075-7096, XP020319397, ISSN: 0031-9155, DOI: 10.1088/1361-6560/AA81F4 [retrieved on 2017-08-11]**
• **SAKAE TAKEJI ET AL: "Multi-layer energy filter for realizing conformal irradiation in charged particle therapy", MEDICAL PHYSICS, AIP, MELVILLE, NY, US, vol. 27, no. 2, 1 February 2000 (2000-02-01), pages 368-373, XP012011057, ISSN: 0094-2405, DOI: 10.1118/1.598840**

**Description**

**FIELD OF THE INVENTION**

The present invention relates to a ridge filter for depositing predefined doses (Dij) over a whole treatment volume (V) by irradiation thereof by beams of accelerated particles (preferably protons) by pencil beam scanning (PBS) in a single painting layer. In particular, the present invention concerns a method for designing such ridge filter, optimizing the dimensions thereof to accurately deposit the doses (Dij) according to a pre-established treatment plan. The ridge filter of the present invention is particularly adapted for flash irradiation of the treatment volume (V) or of a portion thereof by PBS at ultra-high dose deposition rates (HDR).

**BACKGROUND OF THE INVENTION**

Radiation therapy with particles or waves, such as electron beams, protons beams, heavy ions beams, x-rays, $\gamma$-rays, and the like, has become an essential tool for treating patients with tumours.

Since both tumoural cells and healthy cells comprised in a volume are damaged by such radiations, a first challenge in cancer treatment is to define a treatment plan ensuring that defined doses are deposited into the tumoural cells to effectively destroy or kill them, while limiting the doses deposition into healthy cells to spare them as much as possible. A second challenge is to actually deposit the defined doses into the tumoural cells whilst limited doses are actually deposited into the healthy cells, especially those adjacent to the tumoural cells

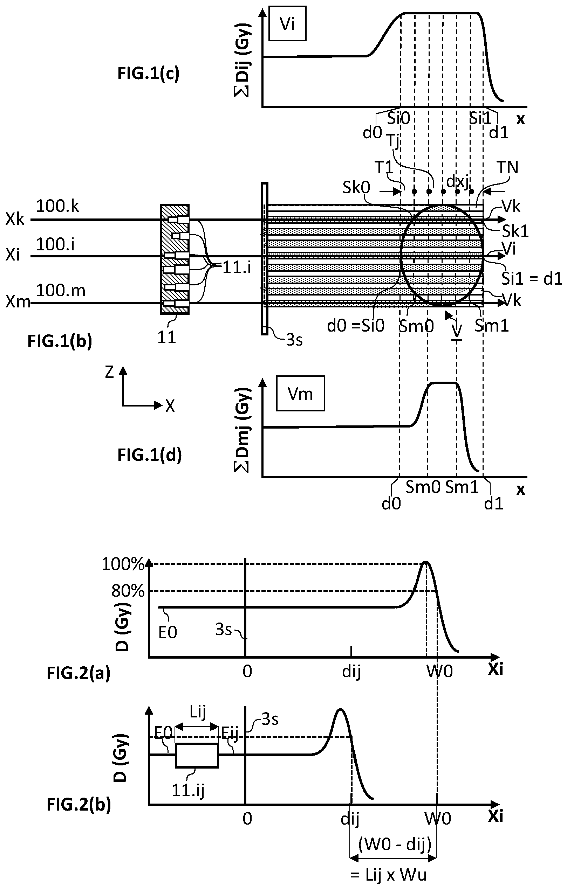

The treatment plan must ensure that, at the end of the treatment, a total target dose sufficient to kill the tumoural cells is delivered into the volume, whilst minimizing the degradation of the healthy cells adjacent to the tumoural cells. Different radiations deposit their energies in different patterns. For example, X-rays deposit most of their energy near the level of the skin, and the deposited energy decreases with penetration depth into the tissues. Healthy tissues located upstream of a target volume of tumoural cells therefore receive a higher dose than the tumoural cells of the target volume. By contrast, as shown in Figures 2(a)&2(b), charged particle beams, in particular protons deposit most of their energy close to the end of their beam path, forming a so-called Bragg peak.

Pencil beam scanning (PBS) is a technique consisting of steering a beam of charged particles along corresponding beam axes (Xi) towards individual spots of a mesh of spots (Sij) defining a target volume comprising tumoral cells. Predefined target doses are thus deposited into cells aligned with the individual spots. The beam is steered along the corresponding beam axes (Xi) and the dose deposition proceeds according to the treatment plan defining the doses (Dij) to be deposited into each cell aligned with a given spot along a beam axis (Xi), as well as the scanning sequence of spots irradiation. PBS reduces unnecessary radiation exposure to surrounding non-cancerous cells by shaping the area being treated to mirror the tumour geometry. Beside the geometry of the target, PBS allows local tuning of the intensity of a beam depending on the position of the spot within the target.

The mesh generally comprises several painting layers (Tj = T1 to TN) normal to an irradiation axis (X) which the beam axes (Xi) are centred upon. Spots (Sij, Si(j+1) ...) are disposed in 2D-arrays on corresponding upstream planes of each layer (Tj). Each painting layer defines a number of cells (Cij, C(i+1)j ...) defined as generalized cylinders with the corresponding spot (Sij) as a base and with generatrixes parallel to the corresponding beam axis (Xi). The cells (Cij) have the same thickness as the corresponding layer (Tj). The superposition of the painting layers (Tj) defines a whole of the treatment volume (V). The spots (Sij) of an upstream layer (Tj) are not necessarily aligned along a corresponding beam axis (Xi) with the corresponding spots (Si(j+1), Si(j+2) ...) of the downstream layers (T(j+1), T(j+2) ...).

The terms *"upstream"* and *"downstream"* are defined relative to the charged particles beam (100.i) direction. Unless otherwise indicated, the terms *"generalized cylinder", "cylinder"* and derivatives thereof refer herein to a surface consisting of all the points on all the lines which are parallel to a generatrix and which pass through a perimeter of a base comprised in a plane not parallel to the generatrix. The base can have any planar geometry. In the special case where the base is circular, it defines a circular cylinder. In case the base is polygonal, it forms a prism. A right cylinder is a cylinder whose base is normal to the generatrix.

PBS is very advantageous because it optimizes the geometrical distribution of the doses deposition to match it with the geometry of the treatment volume (V) enclosing the tumour. PBS can, however, be long as the beam must scan over each spot (Sij) and over each layer (Tj). Moving the beam from a beam axis (Xi) to a different beam axis (X(i+1)) requires a time of a few ms. Changing the energy of a given beam parallel to a given beam axis (Xi) to deposit the desired doses (Dij) into the cells (Cij) of different layers (Tj) is more time consuming and is of the order of 500 ms. The number of layers (Tj) therefore has a strong influence on the duration of a treatment..

With accelerated proton beams, a given beam parallel to a given beam axis (Xi) can successively deposit a predefined charge into the corresponding cells (Cij) of each painting layer aligned along the given beam axis (Xi) by superimposing a number of Bragg peaks staggered in depth at each painting layer along the beam axis (Xi). This results in a Spread Out Bragg Peaks (SOBP) extending over all the cells (Cij, Ci(j+1) ...) aligned along the given beam axis (Xi).

This operation, however, requires successively changing the energy of the given beam such that the corresponding Bragg Peaks be centred on the corresponding cells (Cij). This operation is time consuming. Like in a painting, applying several layers or successively changing the energy of a given beam is time consuming and it would be preferred to be able to deposit all doses (Dij) along an axis beam (Xi) centred on a given spot (Sij) over all the layers with one beam of constant energy, i.e., applying a single paint layer.

Saving treatment time reduces the occupation time of a particle accelerator by each patient. It is also more comfortable for the patient. It is also advantageous if the treatment plan comprises FLASH irradiation, wherein doses are deposited into the cells at ultra-high deposition rates (HDR), of at least 1 Gy / s. A given dose deposited at HDR has shown to spare healthy cells relative to the same dose deposited at lower deposition rates (CDR). Where FLASH irradiation is particularly interesting, is that a given dose deposited into tumoural cells has the same killing effect irrespective of whether it was deposited at HDR or at CDR. Depositing doses (Dij) layer by layer into a treatment volume (V) by PBS would, however, substantially reduce the deposition rates into the cells, since the cells located upstream in the volume (V) are necessarily repeatedly irradiated several times until all cells (Cij) aligned along the given beam axis (Xi) have received their corresponding doses (Dij).

Depositing the predefined doses (Dij) into a treatment volume by PBS with a single layer can be achieved by using a ridge filter. A ridge filter requires the spots (Sij, Si(j+1) ...) of each layer (Tj) to be aligned along corresponding beam axes (Xi). Ridge filters comprising energy degrading units in the form of smooth- or step-pyramids or crests have been described in the art. For example, Simeonov et al., Phys. Med. Biol. 62 (2017) 7075 describe a ridge filter comprising a plurality of energy degrading units in the form of smooth pyramids extending along the beam axes (Xi) corresponding to each spot (Sij). Sakae et al., Med. Phys. 27, 2, (2000) 368 describe a multi-layered ridge filter, the layers being stacked on top of each other, and each layer comprising parallel linear crests having a step-pyramidal cross-section. JP2019136167 describes a ridge filter comprising conical or triangular pyramid protrusions positioned upstream of a Bragg peak expansion filter in the shape of a plate comprising a plurality of through-holes for increasing the width of the SOBP along the corresponding beam axis (Xi).

The principle of a ridge filter is that portions of a beam (100.i) of given energy oriented along a corresponding beam axis (Xi) pass through different material thicknesses of the filter, producing Bragg peaks with different ranges, whose superposition results in a homogeneous SOBP extending over a cylindrical volume defined by a spot (Si1) in a first layer (T1) to a corresponding spot (SiN) in a last layer (TN) downstream of the first layer (T1) spanning a whole depth of the treatment volume (V) along the corresponding beam axis (Xi).

Designing and dimensioning the energy degrading units of a ridge filter remains a challenge. Simeonov et al. describe a complex method for dimensioning the pyramidal protrusions forming the ridge filter, including minimizing the function (DSOBP - D(z))$^2$, wherein DSOBP defines a uniform dose distribution and $D(z) = \sum_{i=1}^{N} \omega i \; x \; B(z + \Delta zi \; x \; i)$ , wherein B(z) is the measured pristine Bragg peak, $\Delta z$ is the step size between the successive Bragg peaks, the weights $\omega i$ determine the contribution of each peak i to the SOBP, and D(z) is the resulting depth dose distribution. The resolution of this equation requires empirical fit-functions. Sakae et al. do not give much information on how to design their ridge filter other that a Monte Carlo calculation is used.

Document "3D range-modulator for scanned particle therapy: development, Monte Carlo simulations and experimental evaluation" (Simeonov Y, Weber U, Penchev P, Ringbæk TP, Schuy C, Brons S, Engenhart-Cabillic R, Bliedtner J, Zink K.; Phys Med Biol. 2017 Aug 11; 62(17):7075-7096) discloses a method for designing a ridge filter of a charged particle accelerator, using pencil beam scanning, spot by spot according to a predefined treatment plan, in a single painting layer defining the whole treatment volume.

A simple, reliable, and reproducible method for dimensioning the energy degrading units of a ridge filter is therefore needed. Alternative ridge filter designs, easier to produce with the required accuracy are proposed. These and other advantages are described in more detail in continuation.

## SUMMARY OF THE INVENTION

The present invention concerns a method for designing a ridge filter of a treatment system comprising a charged particle accelerator, preferably an accelerator of protons, a beamline nozzle, a collimator, and the like, collectively herein referred to as an accelerator, for depositing with beams of accelerated particles specific doses (Dij) into specific locations within a treatment volume of tissue comprising tumoral cells. The present invention concerns deposition by pencil beam scanning (PBS), spot by spot according to a predefined treatment plan (TP), in a single painting layer defining the whole treatment volume. The beams extend along corresponding beam axes (Xi) substantially parallel to an irradiation axis (X), diverging from parallelism from the irradiation axis (X) by an angle comprised within ± 5°, preferably within ± 3°. The tissue is characterized by a maximum beam range (W0), defined as the water equivalent distance at which the beam stops propagating through the tissue. The method comprises the following steps.

The expression *"water equivalent thickness"* (= WET) is well known in the art and is defined as a thickness of water causing a same energy degradation of a particle beam as a given thickness of one or more materials crossed by the particle beam.

A boundary is defined, inscribing the treatment volume by defining areas (Aj) over upstream planes (Y,Z)j of N slices (Tj = T1 - TN) of thickness (dxj), wherein the planes (Y,Z)j are normal to the irradiation axis (X). A shortest water equivalent thickness (d0) and longest water equivalent thickness (d1) to a skin of a patient are defined as the points of the boundary closest to and furthest away from the skin, respectively, measured along the irradiation axis (X).

An array of subvolumes (Vi) is defined, each subvolume extending parallel to the corresponding beam axes (Xi) from the skin of the patient to the corresponding furthest water equivalent thickness, and whose projection onto a plane (Y,Z) normal to the irradiation axis (X) defines an array of spots covering a whole area of a projection of the volume onto the plane (Y,Z).

For each slice (Tj) of the N slices (T1-TN), comprised within a subvolume (Vi), a cell is defined as a portion of the subvolume (Vi) comprised within the corresponding slice (Tj), For each cell of the given subvolume (Vi), a cell water equivalent thickness is determined from the skin to a geometrical centre of the cells. A beam weight ($\omega$ij) is attributed to each cell required for depositing into the cell the specific dose (Dij) according to the TP. The beam weight ($\omega$ij) is proportional to the number of charged particles at the cell water equivalent thickness.

The ridge filter can be designed with a set of energy degrading units, wherein each energy degrading unit is configured for reducing an initial energy (E0) of a corresponding beam of charged particles of beam diameter, coaxial with the corresponding beam axes (Xi) and subvolume (Vi) to reduced energies (Eij), such that the specific doses (Dij) are deposited at the cell water equivalent thicknesses into the corresponding cells comprised within the subvolume (Vi) according to the TP. The energy degrading unit of a given subvolume (Vi) is designed as follows.

For each cell of the subvolume (Vi), a degrading subunit is dimensioned, having a generalized cylindrical geometry of base of area (Aij) normal to the corresponding beam axis (Xi) and of generatrixes of length (Lij) parallel to the corresponding beam axis (Xi). The degrading subunit is made of a material having a subunit water equivalent thickness per unit length (Wu) along the corresponding beam axis (Xi) and wherein the length is determined such that the degrading subunit has a subunit water equivalent thickness (Wij = Wu x Lij) equal to a product of the subunit water equivalent thickness per unit length (Wu) and of the length (Lij). A sum of the subunit water equivalent thickness (Wij) and of the cell water equivalent thickness (dij) is equal to the maximum beam range (W0) (i.e., WO = Wij + dij).

As defined supra, the terms *"generalized cylinder", "cylinder"* and derivatives thereof refer herein to a surface consisting of all the points on all the lines which are parallel to a generatrix and which pass through a perimeter of a base comprised in a plane not parallel to the generatrix. The base can have any planar geometry. In the special case where the base is circular, it defines a circular cylinder. In case the base is polygonal, it forms a prism. A right cylinder is a cylinder whose base is normal to the generatrix.

The area (Aij) of a degrading subunit is determined by equating a normalized beam weight ($\omega$ij/$\Sigma_j\omega_{ij}$) with a ratio of an integral of a fluence (F(y,z)) over the subunit base area (Aij) to the same integral over a base area (Abi) of the degrading unit (11.i) ,

$$\frac{\omega_{ij}}{\Sigma_j\,\omega_{ij}} = \frac{\iint_{Aij} F(y,z).\,dy.\,dz}{\iint_{Abi} F(y,z).\,dy.\,dz} \qquad (1)$$

wherein the fluence F(y,z) is a number of charges per unit area of the beam (100.i) at a position (y,z) of the beam, and wherein the base area (Abi) is equal to a sum of the subunit areas (Aij) (i.e., Abi = $\Sigma_j Aij$).

The N degrading subunits are combined to obtain the energy degrading unit designed for degrading the energy of the beam such as to deposit the required doses (Dij) into the subvolume (Vi). The energy degrading units corresponding to all remaining subvolumes (Vi) can be designed as defined supra.

In a preferred embodiment, the specific doses (Dij) are to be deposited according to the treatment plan at ultra-high dose deposition rate (HDR) into at least a selection of the specific locations within the volume of tissue. An ultra-high dose deposition rate (HDR) is defined as a dose deposition rate, HDR $\geq$ 1 Gy / s.

The spots of the array of spots can be separated from one another by a distance (ds) smaller than or equal to 1.8 times a standard deviation ($\sigma$) of the fluence (Fi(y,z)) of the beam at one single spot (i.e., ds $\leq$ 1.8 $\sigma$), preferably smaller than or equal to 1.5 $\sigma$. In these conditions, the fluence (F(y,z)) of the beam going through the base area (Abi) is approximated to being constant over all values of the planes (Y, Z)j defining the boundary inscribing the volume.

Alternatively, the spots of the array of spots can be separated from one another by a distance (ds) larger than 1.2, preferably larger than 1.5 times a standard deviation ($\sigma$) of the fluence (Fi(y,z)) of the beam at a single spot (i.e., ds > 1.2 $\sigma$), In these conditions, the fluence (Fi(y,z)) of the beam going through the base area (Abi) is approximated to being a Gaussian,

$$F_i(y, z) = A_i . e^{-\left(\frac{(y-y_i)^2}{\sigma_y^2} + \frac{(z-z_i)^2}{\sigma_z^2}\right)},$$

where $(y_i, z_i)$ is the coordinate in the (Y,Z) plane of the position of a maximum $(A_i)$ of the fluence of the spot $(S_i)$ and wherein in the case of a circular spot, then $\sigma_y = \sigma_z = \sigma$.

In a preferred configuration of the present invention, the energy degrading units are in the form of orifices arranged side-by-side according to the array of spots in a support base of thickness $(B_i)$ measured along the beam axis $(X_i)$. Each orifice extends from an aperture opening at a surface of the support base and penetrating to a given depth measured along the corresponding beam axes $(X_i)$. Each energy degrading unit (11.i) is formed by one or more degrading subunits in the form of orifices having a generalized cylindrical geometry of cross-sectional areas $(A_i)$ and extending along the corresponding beam axis $(X_i)$ from the aperture in the support block over lengths $(L_{sij})$, such that $L_{ij} = B_i - L_{sij}$. The degrading subunits as defined supra are arranged within the base area $(A_{bi})$.

In an energy degrading unit of this configuration, at least two subunits can be arranged within the base area $(A_{bi})$ in one of a construction in series in parallel, and a mixed construction.

In the construction in series, the degrading subunits are aligned along the corresponding beam axes $(X_i)$, by order of decreasing lengths $(L_{sij})$, preferably coaxially and with the orifice having the longest length $(L_{si3})$ being positioned at a central position. The subunit base area $(A_{ij})$ of a given degrading subunit is equal to a difference of cross sectional areas $(A_{xij} - A_{xi(j + 1)})$ of the cross-sectional area $(A_{xij})$ between the given degrading unit and the cross-sectional area $(A_{xi(j+1)})$ of the degrading unit circumscribed within the given degrading unit.

In the construction in parallel, the degrading subunits are arranged side-by-side within the base area $(A_{bi})$, either without spaces between two degrading subunits, or with a space between two adjacent degrading subunits.

In the mixed construction both in parallel and in series, three or more degrading subunits are arranged both in series and in parallel. One or more structures formed by two or more degrading subunits aligned in series along the corresponding beam axis $(X_i)$ and, optionally; one or more individual degrading subunits, are arranged side-by-side within the base area $(A_{bi})$.

In an alternative preferred configuration of the present invention, the energy degrading units are in the form of pins arranged side-by-side according to the array of spots and supported on a support base of thickness $(B_i)$ measured along the beam axis $(X_i)$. Each pin extends from the support base along the corresponding beam axes $(X_i)$. In this configuration, each energy degrading unit is formed by one or more degrading subunits having a generalized cylindrical geometry of cross-sectional areas $(A_{ij})$ and extending along the corresponding beam axis $(X_i)$ from the support base over lengths $(L_{sij})$, such that $L_{ij} = B_i + L_{sij}$. These degrading subunits are arranged within the base area $(A_{bi})$.

In an energy degrading unit of this configuration, at least two subunits can be arranged within the base area $(A_{bi})$ in one of a construction in series in parallel, and a mixed construction.

In the construction in series, the degrading subunits are aligned along the corresponding beam axis $(X_i)$, by order of decreasing lengths $(L_{sij})$, preferably coaxially and with the pin having the longest length $(L_{si1})$ being positioned at a central position. The subunit base area $(A_{ij})$ of a given degrading subunit is equal to a difference of cross sectional areas $(A_{xij} - A_{xi(j-1)})$ of the cross sectional area $(A_{xij})$ between the given degrading unit and the cross sectional area $(A_{xi(j-1)})$ of the degrading unit $(A_{xi(j-1)})$ circumscribed within the given degrading unit.

In the mixed construction both in parallel and in series, three or more degrading subunits are arranged both in series and in parallel. One or more structures formed by two or more degrading subunits aligned in series along the corresponding beam axis $(X_i)$ and, optionally; one or more individual degrading subunits, are arranged side-by-side within the base area $(A_{bi})$.

In a preferred embodiment, at least a first degrading subunit of a first energy degrading unit can be made of a first material different from a second material of a second degrading subunit of the first or of a second energy degrading unit. The first material has a value of the subunit water equivalent thickness per unit length $(W_u)$ which is different from the second material, such as to vary, preferably decrease the value of the length $(L_{11} = W_{11} / W_u)$ of the first degrading subunit, compared with the length of a corresponding first energy subunit made of the second material. With the use of different materials of different values of the subunit water equivalent thickness per unit length $(W_u)$, the length $(L_{11})$ of the first degrading subunit can be maintained within $\pm 20\%$ of the length of the second degrading subunit $(L_{ij})$, such as to render the energy degrading unit more compact. The lengths $(L_{ij})$ of all the degrading subunits of an energy degrading unit preferably have a same length $(L_{ij})$ within a variation of $\pm 20\%$ of an average length $(L_{m,ij})$ (i.e., $L_{ij} = L_{m,ij} \pm 20\% \, \forall j$).

The invention is defined in the following claims. Radiotherapy methods are not a part of the invention.

**BRIEF DESCRIPTION OF THE FIGURES**

On these figures,

**Fig. 1(a)** shows schematically a treatment volume (V) divided into subvolumes (Vi) extending parallel to corresponding beam axes (Xi), each subvolume (Vi) being divided into cells (Cij), into which a predefined dose (Dij) is to be deposited. The corresponding SOBP's are illustrated along different beam axes (Xi) passing through corresponding energy degrading units of a ridge filter.

**Fig. 1(b)** shows a side view of the treatment volume (V) of Figure 1(a), with the SOBP's obtained by three beams (100.i, 100.k, 100.m) after passing through a ridge filter according to the present invention.

**Fig. 1(c)** shows the SOBP obtained with the beam (100.i) extending along the beam axis (Xi), which is coaxial with the irradiation axis (X).

**Fig. 1(d)** shows the SOBP obtained with the beam (100.m) extending along the beam axis (Xm).

**Fig. 2(a)** shows the depth dose profile of a typical Bragg peak of a beam of given energy. The abscissa axis represents the depth in water. The maximum beam range (W0) in water is defined as the depth beyond the maximum of the Bragg peak and corresponding to an energy equal to 80% of the maximum of the Bragg peak, wherein WO must be at least equal to or larger than dij (dij < WO).

**Fig. 2(b)** shows the depth dose profile of the Bragg peak of the beam of Figure 2(a) with an energy degrading unit intersecting the path of the beam.

**Fig. 3(a)** shows beams (100.i, 100.(i+1) ...) extending along respective beam axes (Xi, X(i+1) ...) parallel to each other and to the irradiation axis (X) passing through a ridge filter.

**Fig. 3(b)** shows beams (100.i, 100.(i+1) ...) extending along respective beam axes (Xi, X(i+1) ...) fanning out around the irradiation axis (X) passing through a ridge filter.

**Fig. 3(c)** shows schematically a ridge filter comprising energy degrading units in the form of pins.

**Fig. 3(d)** shows two energy degrading units with beams (100.i, 100.(i+1)) extending along respective beam axes (Xi, X(i+1)) which are not parallel to one another ((Xi, X(i+1))-angle exaggerated).

**Fig. 3(e)** shows a volume element of the treatment volume (V) comprising a number of subvolumes (Vi) extending parallel to the irradiation axis (X) and divided into cells (Cij). The SOBP obtained in subvolume (V5) is illustrated.

**Fig. 3(f)** shows a volume element of the treatment volume (V) comprising a number of subvolumes (Vi) extending parallel to the corresponding beam axes (Xi). The beam axes (Xi) are not perfectly parallel to each other nor to the irradiation axis (X). The deviating angles are exaggerated for sake of clarity.

**Fig. 4(a)** illustrates an embodiment of ridge filter comprising energy degrading units formed by gaps.

**Fig. 4(b)** shows a side cut view of a gap forming an energy degrading unit of the ridge filter of Figure 4(a), composed of three degrading subunits. The depth dose profile of the SOBP generated by the degrading unit is represented.

**Fig. 4(c)** shows a side cut view of a first degrading subunit of the energy degrading unit of Figure 4(b). Two examples of possible cross-sections of the first degrading units are shown: circular or polygonal (hexagonal) The Bragg peak generated by the first degrading unit has a maximum at depth di1

**Fig. 4(d)** shows a side cut view of a second degrading subunit of the energy degrading unit of Figure 4(b). Two examples of possible cross-sections of the first degrading units are shown: circular or polygonal (hexagonal). The Bragg peak generated by the first degrading unit has a maximum at depth di2

**Fig. 4(e)** shows a side cut view of a third degrading subunit of the energy degrading unit of Figure 4(b). Two examples of possible cross-sections of the first degrading units are shown: circular or polygonal (hexagonal). The Bragg peak generated by the first degrading unit has a maximum at depth di3.

**Fig. 5(a)** illustrates an embodiment of ridge filter comprising energy degrading units formed by protruding pins.

**Fig. 5(b)** shows a side cut view of a protruding pin forming an energy degrading unit of the ridge filter of Figure 5(a), composed of three degrading subunits. The depth dose profile of the SOBP generated by the degrading unit is represented.

**Fig. 5(c)** shows a side cut view of a first degrading subunit of the energy degrading unit of Figure 5(b). Two examples of possible cross-sections of the first degrading units are shown: circular or polygonal (hexagonal). The Bragg peak generated by the first degrading unit has a maximum at depth di3

**Fig. 5(d)** shows a side cut view of a second degrading subunit of the energy degrading unit of Figure 5(b). Two examples of possible cross-sections of the first degrading units are shown: circular or polygonal (hexagonal). The Bragg peak generated by the first degrading unit has a maximum at depth di2

**Fig. 5(e)** shows a side cut view of a third degrading subunit of the energy degrading unit of Figure 5(b). Two examples of possible cross-sections of the first degrading units are shown: circular or polygonal (hexagonal). The Bragg peak generated by the first degrading unit has a maximum at depth di1

**Fig. 6(a)** shows the side cut view of the energy degrading unit (11.i) of Figure 4(b) comprising N = 3 degrading units (11.ij) formed by sequential gaps and corresponding SOBP.

**Fig. 6(b)** shows a variation of the energy degrading unit (11.i) of Figure 6(a), comprising N = 6 degrading subunits (11.ij) and corresponding SOBP.

**Fig. 6(c)** shows a variation of the energy degrading units (11.i) of Figures 6(a) and 6(b), comprising $N \rightarrow \infty$ degrading subunits (11.ij), forming a continuous truncated generalised conical gap and corresponding SOBP.

**Fig. 6(d)** shows an energy degrading unit (11.i) in the form of a stepped pin, comprising N = 6 degrading subunits (11.ij) and corresponding SOBP.

**Fig. 6(e)** shows a variation of the energy degrading units (11.i) of Figures 6(d), comprising $N \rightarrow \infty$ degrading subunits (11.ij), forming a continuous truncated generalised cone and corresponding SOBP.

**Fig. 7(a)** shows the shift of the Bragg peaks characterizing the dose deposition by a beam passing through an energy degrading unit in the form of a protruding pin comprising two degrading subunits of different lengths and cross-sectional areas and arranged coaxially.

**Fig. 7(b)** shows the shift of the Bragg peaks characterizing the dose deposition by a beam passing through an energy degrading unit in the form of a gap comprising two degrading subunits of different lengths and cross-sectional areas.

**Fig. 7(c)** shows the shift of the Bragg peaks characterizing the dose deposition by a beam passing through an energy degrading unit in the form of concentric elements comprising two degrading subunits of different densities and cross-sectional areas (density of the inner element lower than the density of the peripheral element).

**Fig. 7(d)** shows the shift of the Bragg peaks characterizing the dose deposition by a beam passing through an energy degrading unit in the form of concentric elements comprising two degrading subunits of different densities and cross-sectional areas (density of the inner element larger than the density of the peripheral element).

**Fig. 7(e)** shows the shift of the Bragg peaks characterizing the dose deposition by a beam passing through an energy degrading unit in the form of a protruding pin comprising two degrading subunits of different lengths and cross-sectional areas and arranged side-by-side.

**Fig. 7(f)** shows a perspective view of an energy degrading unit (11.i) of the type illustrated in Figure 7(a).

**Fig. 7(g)** shows a perspective view of an energy degrading unit (11.i) of the type illustrated in Figure 7(e).

**Fig. 7(h)** shows a perspective view of an embodiment of an energy degrading unit (11.i) comprising degrading subunits arranged both in series and in parallel in a mixed configuration.

**Fig. 7(i)** shows a perspective view of an alternative embodiment of an energy degrading unit (11.i) comprising degrading subunits arranged both in series and in parallel in a mixed configuration.

**DETAILED DESCRIPTION.**

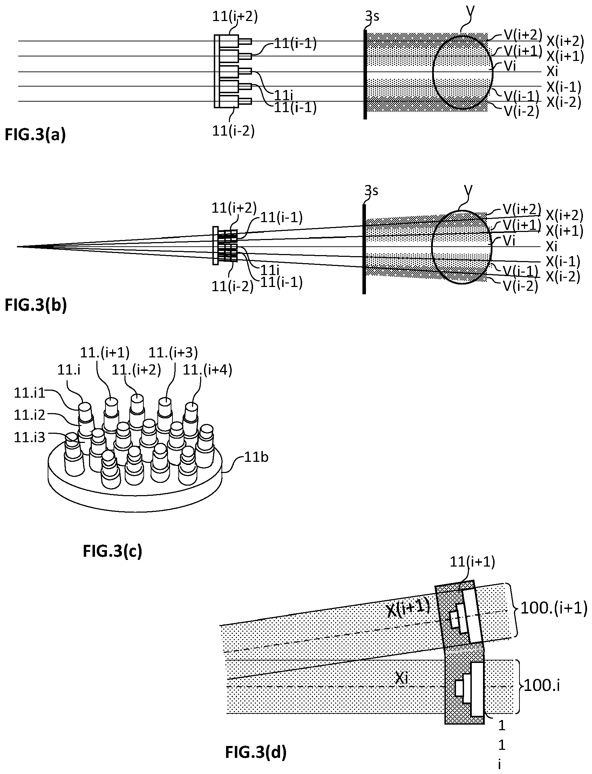

The present invention concerns a method for designing a ridge filter (11) of a charged particles accelerator, preferably an accelerator of protons. The ridge filter (11) of the present invention is configured for depositing with beams of accelerated particles (100.i) specific doses (Dij) into specific locations within a treatment volume (V) of tissue comprising tumoral cells (3t) according to a predefined treatment plan (TP) by pencil beam scanning (PBS), spot by spot (Si) of an array of spots. The ridge filter allows the PBS to be carried out in a single painting layer defining the whole treatment volume (V). In PBS, a narrow pencil beam is deflected to scan each spot (Si) of the array. Although it is a single beam that is being deflected, in continuation each beam (100.i) aimed at a corresponding spot (Si) extending along a corresponding beam axis (Xi) is treated as an individual beam different from a beam (100.k) aimed at a different spot (Sk, k ≠ i) and extending along a second beam axis (Xk). As illustrated in Figures 1(b) and 3(a), the beam axes (Xi) are substantially parallel to an irradiation axis (X). In practice, since the "individual" beams (100.i) are a single beam which is being deflected from a common accelerator outlet point towards the individual spots (Si) of the array, the beam axes (Xi) diverge from parallelism with the irradiation axis (X) by an angle comprised within ± 5°, preferably within ± 3°, depending inter alia on the size of the treatment volume (V) and on the distance separating the accelerator outlet from the treatment volume (V).

The tissue traversed by the beams (100.i) absorbs a fraction of the energy of the beams determining the penetration depth of the Bragg peak position along the beam axis (Xi). The penetration depth of the Bragg peak position in a given tissue can be characterized by a maximum beam range (W0) in water, defined as the *"water equivalent thickness"* (= WET), i.e., defining the position where the beam stops propagating in water. The expression *"water equivalent thickness"* (= WET) is defined as a thickness of water causing a same energy degradation of a particle beam as a given thickness of one or more materials crossed by the particle beam. The maximum beam range (W0) can be related directly to penetration depth of the same beam through the tissue. It follows that WET and penetration depth of the Bragg peak position through a tissue can be used interchangeably, the former (WET) being of course easier to test and measure experimentally.

The method for designing a ridge filter (11) according to the present invention comprises the following steps:

• Defining the treatment volume (V) and the cartography indicating the positions and doses to be deposited therein, and

• Designing and dimensioning the ridge filter accordingly.

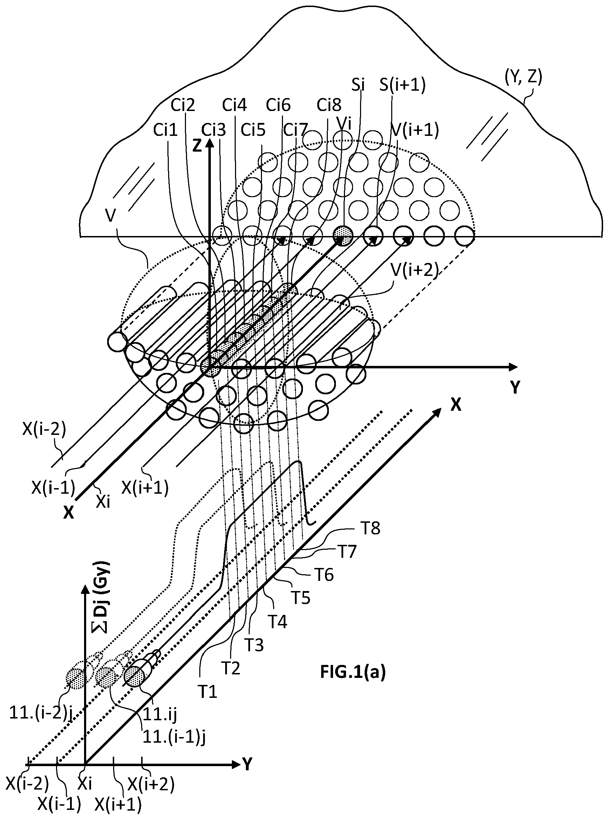

The definition of the treatment volume includes first, as illustrated schematically in Figures 1(a), 1(b), 3(a), and 3(b), defining a boundary inscribing the treatment volume (V). This operation includes defining areas over upstream planes (Y,Z)j of N slices (Tj = T1 - TN) of thickness (dxj), wherein the planes (Y,Z)j are normal to the irradiation axis (X). An upstream plane (Y,Z)j is the plane (Y,Z) of a slice (Tj) which is first hit by the beam (100.i). The depth of the treatment volume (V) measured along the irradiation axis (X) is comprised between a shortest water equivalent thickness (d0) and longest water equivalent thickness (d1) measured from a skin (3s) of a patient, which correspond to the points of the boundary closest to and furthest away from the skin (3s), respectively, measured along the irradiation axis (X).

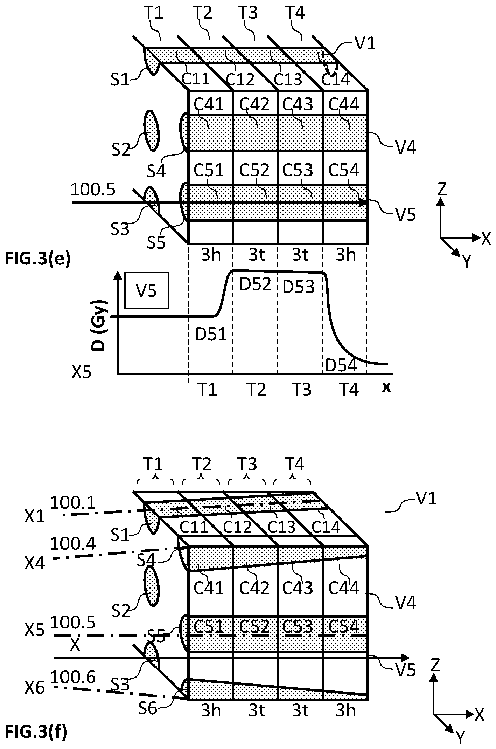

Second, as illustrated in Figures 1(a), 1(b), 3(e), and 3(f), the treatment volume (V) inscribed within the boundary is divided by defining an array of subvolumes (Vi). Each subvolume (Vi) extends parallel to the corresponding beam axes (Xi) from the skin of the patient to the corresponding furthest water equivalent thickness (d1), and whose projection onto a plane (Y,Z) normal to the irradiation axis (X) defines an array of spots (Si) covering a whole area of a projection of the volume (V) onto the plane (Y,Z) (cf. Figure 1(a)).

Third, the subvolumes (Vi) are themselves divided into cells (Cij) as follows. For each slice (Tj) of the N slices (T1-TN), comprised within a subvolume (Vi), a cell (Cij) is defined as a portion of the subvolume (Vi) comprised within the corresponding slice (Tj), This is illustrated in Figures 1(a), 3(a), and 3(b). For each cell (Cij) of the given subvolume (Vi), a cell water equivalent thickness (dij) is determined from the skin (3s) to a geometrical centre of the cells (Cij). A beam weight (ωij) is attributed to each cell (Cij) required for depositing into the cell (Cij) the specific dose (Dij) according to the TP. The beam weight (ωij) corresponds to the number of charged particles (e.g., protons) to be delivered within a spot (Cij). The beam weight (ωij) is therefore proportional to the number of charged particles at the cell water equivalent thickness (dij).

Once the treatment volume has been divided into subvolumes (Vi) and cells (Cij), and once the beam weights (ωij) required for depositing the doses (Dij) into each cell (Cij) according to the treatment plan have been determined, the ridge filter (11) can be designed and dimensioned accordingly as follows.

The ridge filter (11) comprises a set of energy degrading units (11.i) configured for reducing an initial energy (E0) of a beam (100.i) of charged particles of beam diameter (D100.i), coaxial with the corresponding beam axes (Xi) and subvolume (Vi) to reduced energies (Eij), such that the specific doses (Dij) are deposited at the cell water equivalent thicknesses (dij) into the corresponding cells (Cij) comprised within the subvolume (Vi) according to the TP. The principle is illustrated in Figures 2(a) and 2(b).

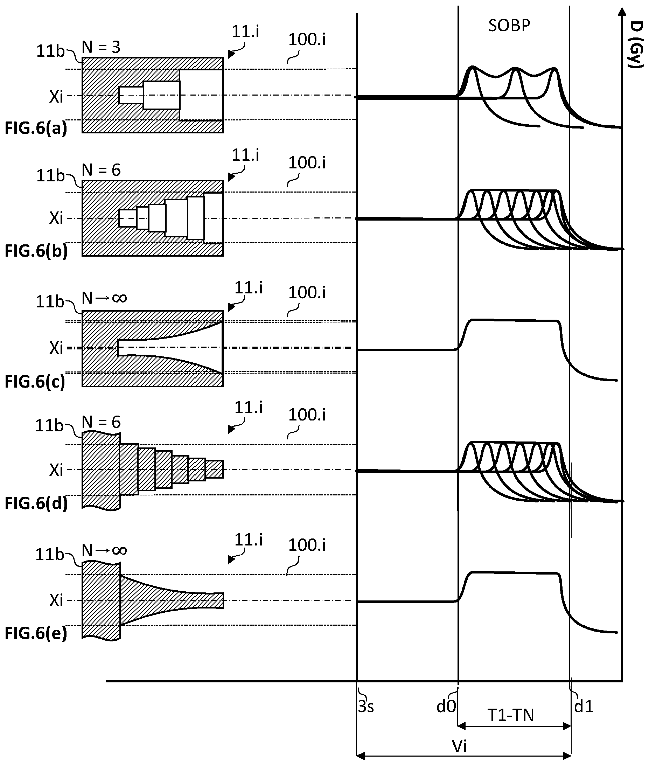

Figure 2(a) plots the dose (Dij) deposited as a function of the WET along a beam axis (Xi) by a beam (100.i) of given energy (E0). As discussed above, the maximum beam range in water (WET) is defined as WO, corresponding to the depth beyond the maximum of the Bragg peak and corresponding to an energy equal to 80% of the maximum of the Bragg peak. This means that the beam (100.i) of given energy cannot penetrate deeper into the tissues than the corresponding water equivalent thickness WO (i.e., d1 ≤ WO). If d1 > WO, i.e., the treatment volume (V) extends deeper than the Bragg peak, then a beam of higher energy must be used. Figure 2(b) shows the displacement of the Bragg peak upon inserting a degrading subunit (11.ij) across the path of the same beam (100.i) of given energy. It can be seen that the WET of the Bragg peak is now at a depth (dij < WO) from the skin (3s). This is because a portion (E0 - Eij) of the energy of the beam (100.i) is absorbed by the degrading subunit (11.ij) the beam must cross. Figure 2(b) shows how to displace one Bragg peak form a WET = WO to a desired WET = dij by interposing a degrading subunit (11.ij). Since a SOBP can be formed by superimposing several Bragg peaks distributed over a given depth along the irradiation axis (X), to deposit the required doses (Dij) into a whole subvolume (Vi) by PBS with a single painting layer, several degrading subunits (11.ij) can be superimposed to form an energy degrading unit (11i), wherein each degrading subunit (11.ij) is dimensioned such as to shift the Bragg peak from WET = WO to a corresponding cell equivalent thickness (dij) to ensure that the required doses (Dij) be deposited in each cell (Cij) of the subvolume (Vi) irradiated by the beam (100.i). This is illustrated in Figures 6(a) to 6(e), showing energy degrading units (11.i), each composed of N = 3, N = 6, and N→∞ degrading subunits (11.ij) with the corresponding SOBP. The number of Bragg peaks and their corresponding cell water equivalent thicknesses (dij) required for yielding the desired SOBP according to the TP must be determined. The number of Bragg peaks in a given beam (100.i) and, therefore, of degrading subunits (11.ij) of a corresponding energy degrading unit (11.i) is equal to the number N of slices (T1-TN). Each Bragg peak of the given beam (100.i) has a corresponding energy degrading unit (11.i) whose degrading subunits (11.ij) can be designed to yield the required SOBP and thus deposit the required doses (Dij) into the corresponding cells (Cij). When N→∞ degrading subunits (11.ij) as illustrated in Figures 6(c) and 6(e), the steps of the stepped configuration of the energy degrading unit (11.i) become smaller until tending to zero, yielding the smooth geometry of a truncated generalized cone illustrated in Figures 6(c) and 6(e), wherein a generalized cone can have a base of any geometry, not restricted to a circle.

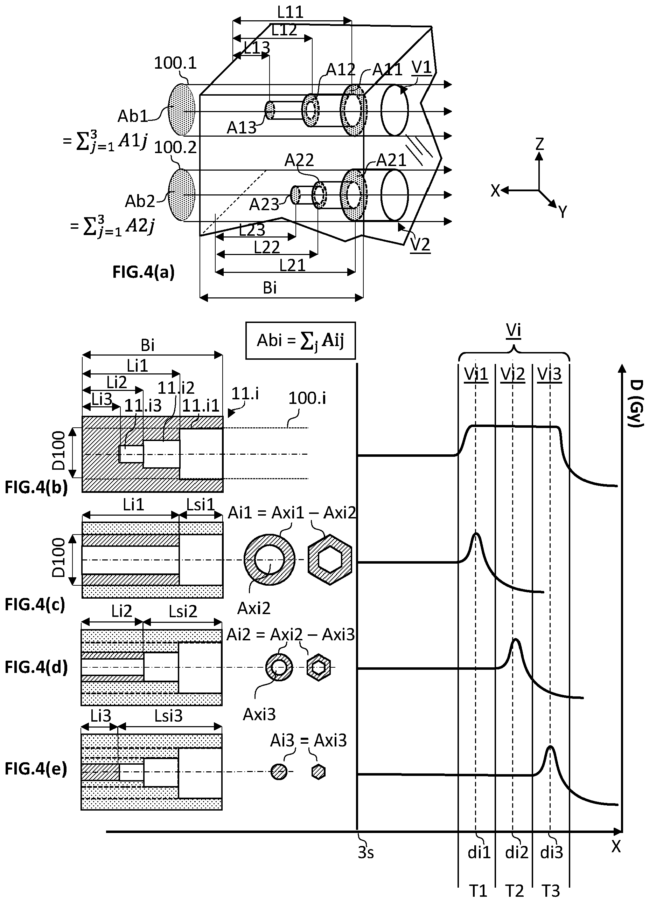

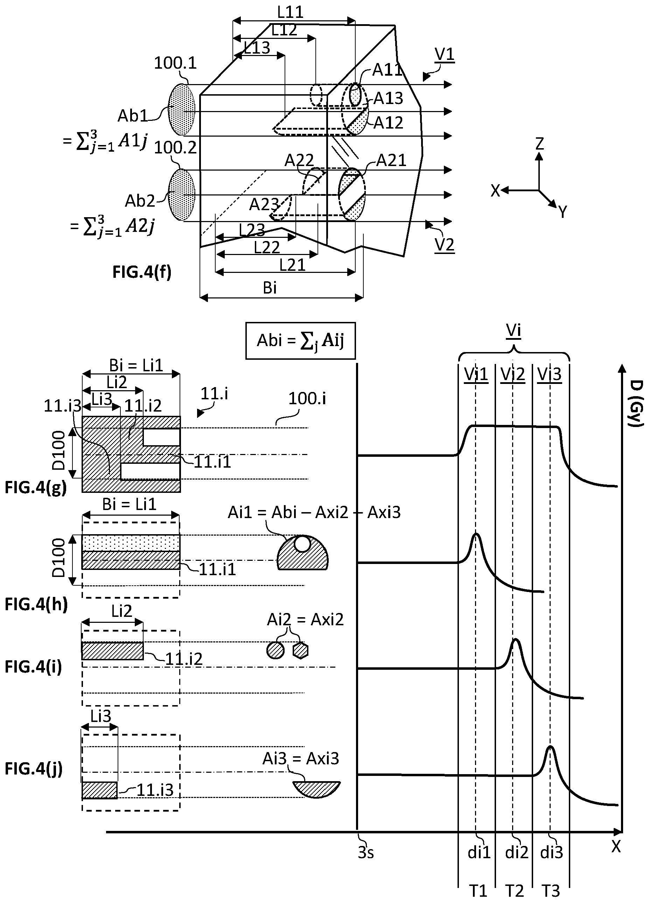

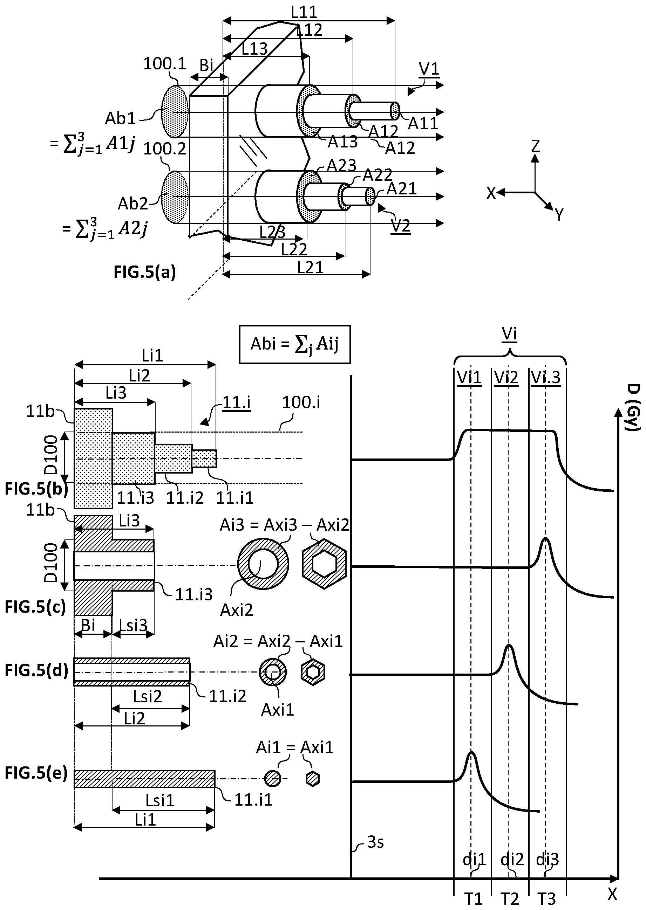

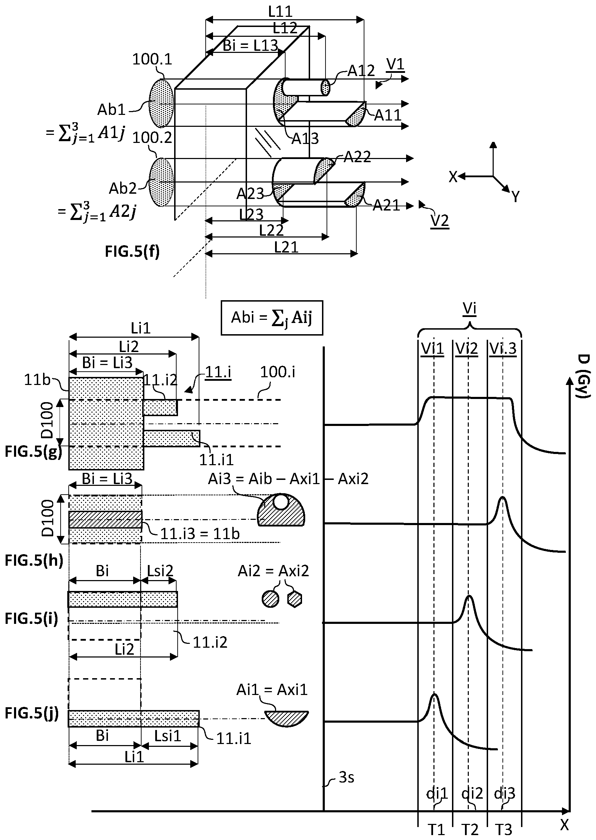

Each degrading subunit (11.ij) has a generalized cylindrical geometry (i.e., not necessarily a circular cylinder) of base of area (Aij) normal to the corresponding beam axis (Xi) and of generatrixes of length (Lij) parallel to the corresponding beam axis (Xi). The degrading subunits illustrated in Figures 4(a) to 4(e) and 5(a) to 5(e) have a circular or polygonal cross-section. It is clear that other cross-sections are possible. A certain degree of symmetry of the cross-sections is, however, preferred to facilitate the calculation of the dimensioning of the areas (Aij). The degrading subunits are made of materials having a subunit water equivalent thickness per unit length (Wu) along the corresponding beam axis (Xi). The length (Lij) and area (Aij) of each degrading subunit (11.ij) can be dimensioned as follows.

The length (Lij) of a degrading subunit (11.ij) is determined such that the degrading subunit (11.i) has a subunit water equivalent thickness (Wij = Wu x Lij) equal to a product of the subunit water equivalent thickness per unit length (Wu) and of the length (Lij). Considering that a sum of the subunit water equivalent thickness (Wij) and of the cell water equivalent thickness (dij) must be equal to the maximum beam range (W0) (i.e., WO = Wij + dij), it follows that the length (Lij) of a degrading subunit (11.ij) is defined as, $Lij = \frac{1}{Wu}(WO - dij)$, wherein the factor (WO - dij) is illustrated graphically in Figure 2(b). The length of a degrading subunit is thus fully defined.

The area (Aij) of a degrading subunit (11.ij) is determined by equating a normalized beam weight ($\omega ij / \Sigma_j \omega_{ij}$) with a ratio of an integral of a fluence (F(y,z)) over the subunit base area (Aij) to the same integral over a base area (Abi) of the degrading unit (11.i),

$$\frac{\omega_{ij}}{\Sigma_j \omega_{ij}} = \frac{\iint_{Aij} F(y,z).dy.dz}{\iint_{Abi} F(y,z).dy.dz} \qquad (1)$$

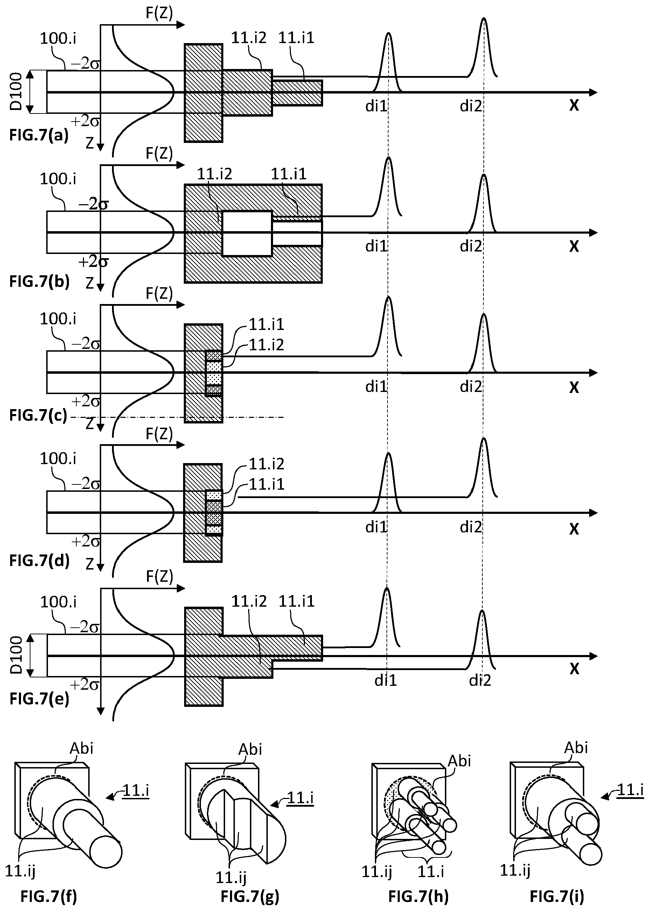

wherein the fluence F(y,z) is a number of charges per unit area of the beam (100.i) at a position (y,z) of the beam, and wherein the base area (Abi) is equal to a sum of the subunit areas (Aij) (i.e., Abi = $\Sigma_j Aij$) as illustrated in Figures 4(a) and 5(a). As illustrated in Figures 7(a) to 7(e) the fluence of a beam of charged particles generally has a Gaussian distribution. The diameter (D100) of a beam can be defined as the distance $4\sigma$, wherein $\sigma$ is the standard deviation of the Gaussian distribution characterizing the fluence of the beam. Approximately 95% of the protons of the spots are located within this diameter of $4\sigma$.

As illustrated in Figures 4(b) to 4(e), 4(g) to 4(j), 5(b) to 5(e), and 5(g) to 5(j), with three degrading subunits (11.ij) of an energy degrading unit (11.i) being dimensioned, they can be combined coaxially to obtain the energy degrading unit (11.i) designed for degrading the energy of the beam (100.i) such as to deposit the required doses (Dij) into the subvolume (Vi).

The same exercise is repeated to design the energy degrading units (11.i) corresponding to all remaining subvolumes (Vi) as defined supra.

## THE ARRAY OF SPOTS (Si)

As illustrated in Figure 1(a), an array of spots (Si) is defined covering an area of a projection parallel to the irradiation axis (X) of the treatment volume (V) onto a projection plane (Y,Z) normal to the irradiation axis (X).

An oncologist characterizes the geometry and topography of the tumour region based on images of the tumour region obtained by computed tomography scan (= CT-scan). As shown in Figure 1(b), to reach the treatment volume (V) a beam (100.i) must cross through healthy cells separating the treatment volume (V) from the skin (3s) of the patient, thus irradiating both healthy cells and tumoural cells. The oncologist defines a treatment plan defining the doses (Dij) to be deposited into the treatment volume (V) as well as the doses depositions not to be exceeded in the healthy cells located outside (in particular upstream) of the treatment volume.

The spots have a dimension normal to the irradiation axis (X), which can be equal to the beam diameter discussed supra. The distance between adjacent spots, defining the array density, is an important parameter, since the denser the array (i.e., the closer adjacent spots are from one another) the more substantial is the effect of overlapping doses to the cells spanned by adjacent spots. A substantial overlap leading to a uniform lateral dose distribution is observed at distances between adjacent spots of about 1.5 $\sigma$.

In a first embodiment, the spots (Si) of the array of spots are separated from one another by a distance (ds) smaller than or equal to 1.8 times the standard deviation ($\sigma$) of the fluence (Fi(y,z)) of the beam (100.i) at one single spot (i.e., ds $\leq$ 1.8 $\sigma$), preferably smaller than or equal to 1.5 $\sigma$. With this configuration, a given subvolume (Vi) receives doses (Dij) from the beam (100.i) centred on the corresponding beam axis (Xi) but also from neighbouring beams centred on adjacent beam axes and whose fluence extends over the given subvolume (Vi) and also extends over the given degrading unit 11.i. Taking account of the doses deposited into the subvolumes (Vi) by adjacent beams, the fluence (F(y,z)) of the beams (100.i) going through the base area (Abi) (cf. Equation (1)) is approximated to being constant over all values of the planes (Y, Z)j of the slices (Tj) defining the boundary inscribing the volume (V).

In a second embodiment, the spots (Si) of the array of spots are separated from one another by a distance (ds) larger than 1.2, preferably larger than 1.5 times a standard deviation ($\sigma$) of the fluence (Fi(y,z)) of the beam (100.i) at a single spot (100.i) (i.e., ds > 1.2 $\sigma$). At such distance, the fluences of adjacent beams through a given degrading unit (11.i) is negligible. The fluence (Fi(y,z)) of the beam (100.i) going through the base area (Abi) is therefore approximated to being a Gaussian,

$$F_i(y,z) = A_i . e^{-\left(\frac{(y-y_i)^2}{\sigma_y^2} + \frac{(z-z_i)^2}{\sigma_z^2}\right)},$$

where (yi,zi) is the coordinate in the (Y,Z) plane of the position of a maximum (Ai) of the fluence of the spot (Si) and wherein in the case of a circular spot, then $\sigma_y = \sigma_z = \sigma$.

Increasing the density of the array of spots (Si) may seem to always be advantageous over a low-density array. Irradiating a high-density array, however, prolongs the scanning time required to cover the whole treatment volume (V). Furthermore, FLASH deposition wherein doses (Dij) are to be deposited at HDR to yield a FLASH-effect to spare the healthy cells, is more difficult to yield with a high-density array because of doses being deposited from adjacent beams, thus prolonging the deposition time (and decreasing the deposition rate accordingly). The density of the array of spots (Si) is therefore to be determined case by case.

## DEGRADING UNITS (11.i)

The principle of the ridge filter (11) of the present invention is for each spot (Si) to degrade the energy of the beam (100.i) such as to yield a desired SOBP in the corresponding subvolume (Vi), with an extended peak extending between the shortest water equivalent thickness (d0) and longest water equivalent thickness (d1) measured from the skin (3s) of the patient, bounding the portion of the subvolume (Vi) comprised within the treatment volume. The challenge is to degrade the energy of only a fraction of the beam (100.i) such as to move the Bragg peaks of predetermined beam weight fractions ($\omega$ij) of the whole weight ($\omega$i) of the beam (100.i) to the corresponding cell water equivalent thicknesses (dij), so that the desired doses (Dij) be deposited into the corresponding cells (Cij) of the corresponding suvolumes (Vi).

Three geometries of energy degrading units (11i) are proposed here to achieve this goal, with corresponding methods for dimensioning the degrading subunits (11.ij) forming the energy degrading units (11i). A geometry can be combined with another to achieve the most convenient ridge filter.

• energy degrading units (11i) in the form of orifices,

• energy degrading units (11i) in the form of pins,

• energy degrading units (11i) combining different materials having different subunit water equivalent thicknesses per unit length (Wu).

## DEGRADING UNITS (11.i) = ORIFICES

In this embodiment, illustrated in Figures 4(a) to 4(e), 4(f) to 4(j), and 6(a) to 6(c), the energy degrading units (11.i) are in the form of orifices arranged side-by-side according to the array of spots (Si) in a support base (11b) of thickness (Bi) measured along the beam axis (Xi). Each orifice extends from an aperture opening at a surface of the support base (11b) and penetrates to a given depth measured along the corresponding beam axes (Xi). Each energy degrading unit (11.i) is formed by one or more degrading subunits (11.ij, 11.i3, 11.i2, 11.i1) in the form of orifices having a generalized cylindrical geometry of cross-sectional areas (Axij), and extending along the corresponding beam axis (Xi) from the aperture in the support block (11b) over lengths (Lsij), such that Lij = Bi - Lsij. The degrading subunits (11.ij, 11.i3, 11.i2, 11.i1) are arranged within a diameter slightly larger than or equal to the diameter (D100) of the beams (100.i) and such that the sum of the subunit base areas (Aij) is equal to the base area (Abi) (i.e., $\sum_{j=1}^{N} Aij = Abi$ ).

In a preferred embodiment illustrated in Figures 4(a) to 4(e) and 7(b), the degrading subunits are arranged in a construction in series, wherein the degrading subunits (11.ij) are aligned along the corresponding beam axis (Xi), by order of decreasing lengths (Lsij). The degrading subunits (11.ij) are preferably arranged coaxially and with the orifice having the longest length (Lsi3) being positioned at a central position. They can also be arranged in other configurations, e.g., not concentrically, and with the orifice of longest length (Li3) not being necessarily centred.

In the embodiment wherein the degrading subunits (11.ij) are arranged concentrically, the subunit base area (Aij) of each degrading subunit (11 .ij) with the exception of the subunit base area (Ai3) of deepest orifice (11.i3) has an annular geometry. Consequently, the subunit base area (Aij) of a given degrading subunit (11.ij) is equal to a difference of cross sectional areas (Axij - Axi(j + 1)) of the cross-sectional area (Axij) between the given degrading unit (11.ij) and the cross-sectional area (Axi(j+1)) of the degrading unit (Axi(j+1)) circumscribed within the given degrading unit, wherein the cross-sectional area (Axij) of a degrading subunit (11 .ij) is the cross-sectional area of an orifice normal to the corresponding beam axis (Xij) comprised within an outer perimeter of the cross-section of the degrading subunit (11.ij).

In an alternative embodiment illustrated in Figures 4(f) to 4(j), the degrading subunits can be arranged in a construction in parallel. In this embodiment, the degrading subunits are arranged side-by-side within the base area (Abi), either without spaces between two degrading subunits as illustrated for pins in Figures 7(e) and 7(g) (it is easy to imagine the corresponding design with orifices), or with a space between two adjacent degrading subunits as shown in Figures 4(f) and 4(g),

In yet an alternative embodiment, the degrading subunits can be arranged in a series and parallel mixed construction. In this construction, illustrated for pins in Figures 7(h) and 7(i) (it is easy to imagine the corresponding design with orifices), three or more degrading subunits (11.ij) are arranged both in series and in parallel, with one or more structures formed by two or more degrading subunits aligned in series along the corresponding beam axis (Xi) and, optionally; one or more individual degrading subunits, are arranged side-by-side within the base area (Abi).

This embodiment is particularly easy to produce, either by machining or etching a block forming the support base (11b) or by forming it by a 3D-printing technique. Considering the maximum fluence of the beam (100.i) is at the beam axis (Xi), errors in manufacturing the degrading subunit (11.ij) intersecting the beam axis (Xi) at the maximum of the Gaussian distribution of the fluence are therefore more critical than at peripheral positions. Using orifices rather than pins has the advantage that the weight attributed to the Bragg peak matches the fluence of the spots. Indeed, the Bragg peak with the larger range is usually the one with the larger weight ($\omega ij$) in the treatment plan. Therefore, it is better to place the corresponding subunit (i.e. with the smallest length Li) at the location of the highest fluence of the beam (i.e. in the centre). In addition, the subunit with the largest length Li will correspond to the Bragg peak (with the shortest range) with usually a small weight and therefore a small cross section. It is therefore convenient that it be located in the region of the spot with the smallest fluence. Forming an orifice with tight tolerances is easier than forming a thin pin of length (Lij) with the same tolerances, required to yield the desired effect. Offsetting the degrading subunit of longest length (Lij) can also contribute to decreasing the weight of slight deviations in manufacturing.

For sake of clarity, Figures 4(a) to 4(e) show an energy degrading unit (11.i) formed of three degrading subunits (11.ij) and Figure 7(b) show an energy degrading unit (11.i) formed of two degrading subunits (11.ij) only. It is clear that the number (N) of degrading subunits can in practice be higher to yield a smoother SOBP plateau. Figures 6(a) to 6(c) show similar energy degrading units (11.i) comprising three, six, an "infinite number" of degrading subunits (11.ij), the latter forming a trunco-conical orifice. All orifices in Figures 6(a) to 6(c) have an opening area (Axi1) and a bottom area (Axi3), and of length (Lsi3) (cf. Figures 4(a) to 4(e) for the meaning of the symbols).

In Figures 4(c) to 4(e), each degrading subunit is illustrated separately and, in its entirety forming generalized hollow cylinders, which are fitted into one another to form the degrading subunit of Figure 4(b). In practice, the degrading subunit (11.ij) of Figure 4(b) can be produced either by machining or etching a support base (11b) to form the orifices or by forming the ridge filter by 3D-printing techniques, with corresponding orifices.

## DEGRADING UNITS (11.i) = PINS

In an alternative embodiment illustrated in Figures 5(a) to 5(e), 7(a) 7(e), and 7(f), the energy degrading units (11.i) are in the form of pins arranged side-by-side according to the array of spots (Si) and supported on a support base (11b) of thickness (Bi) measured along the beam axis (Xi). Each pin extends from the support base along the corresponding beam axes (Xi). Each energy degrading unit (11.i) is formed by one or more degrading subunits (11.ij, 11.i3, 11.i2, 11.i1) having a generalized cylindrical geometry of cross-sectional areas (Axij), and extending along the corresponding beam axis (Xi) from the support base over lengths (Lsij), such that Lij = Bi + Lsij. The degrading subunits (11.ij, 11.i3, 11.i2, 11.i1) are arranged within a diameter slightly larger than or equal to the diameter (D100) of the beams (100.i) and such that the sum of the subunit base areas (Aij) is equal to the base area (Abi) (i.e., $\sum_{j=1}^{N} Aij = Abi$).

In a preferred embodiment illustrated in Figures 5(a) to 5(e) and 7(a), the degrading subunits (11.ij) are arranged in series. In this construction, the degrading subunits are aligned along the corresponding beam axis (Xi), by order of decreasing lengths (Lsij). They are preferably arranged coaxially and with the pin having the longest length (Lsi1) being positioned at a central position.

In the embodiment wherein the degrading subunits (11.ij) are arranged concentrically, the subunit base area (Aij) of a given degrading subunit (11.ij) is equal to a difference of cross sectional areas (Axij - Axi(j - 1)) of the cross-sectional area (Axij) between the given degrading unit (11.ij) and the cross-sectional area (Axi(j-1)) of the degrading unit (Axi(j-1)) circumscribed within the given degrading unit. In other words, for a finite number N of degrading subunits (11.ij), the pins are in the shape of stepped pyramids (cf. Fiures 4(b) to 4(e)). The area (Aij) of a given degrading subunit (11.ij) is the area of the tread (or flange) of the step formed by the given degrading subunit (11.ij), surrounding the next degrading subunit (11.i(j+1)) of smaller dimensions than the given degrading subunit (11.ij).

In an alternative embodiment illustrated in Figures 5(f) to 5(j), 7(e), and 7(g), the degrading subunits can be arranged in construction in parallel. In this embodiment, the degrading subunits are arranged side-by-side within the base area (Abi), either without spaces between two degrading subunits as illustrated in Figures 7(e) and 7(g), or with a space between two adjacent degrading subunits as shown in Figures 5(f) and 5(g), For example, in the embodiment illustrated in Figures 7(e) and 7(g), the degrading subunits are arranged side-by-side, with the longest degrading subunit being preferably offset relative to the corresponding beam axis (Xi). This way, the longest degrading unit faces a portion of the beam (100.i) with lower weight and the cross-sectional areas (Aij) can therefore be larger, thus facilitating the manufacturing of the energy degrading unit (11.i). The same configuration can also be applied to energy degrading units (11.i) in the form of orifices, discussed supra.

In yet an alternative embodiment, three or more degrading subunits can be arranged both in series and in parallel in a mixed construction. In this construction, illustrated in Figures 7(h) and 7(i), three or more degrading subunits (11.ij) are arranged both in series and in parallel, with one or more structures formed by two or more degrading subunits aligned in series along the corresponding beam axis (Xi) and, optionally; one or more individual degrading subunits, are arranged side-by-side within the base area (Abi).

For sake of clarity, Figures 5(a) to 5(e) and 7(f) show an energy degrading unit (11.i) formed of three degrading subunits (11.ij) and Figures 7(a) and 7(e) show energy degrading units (11.i) formed of two degrading subunits (11.ij) only. It is clear that the number (N) of degrading subunits can in practice be higher to yield a smoother SOBP plateau. As illustrated in Figures 5(b), 6(d) and 6(e), the energy degrading units (11.i) in the form of pins can comprise any number, such as three, six, an "infinite number" of degrading subunits (11.ij), the latter forming a truncated cone (cf. Figure 6(e)). Regardless of the number N of degrading subunits (11.ij), all pins have a base area (Ai1) and a tip area (Ai3), and of length (Lsi3) (cf. Figures 5(a) to 5(e) for the meaning of the symbols).

In Figures 5(c) to 5(e), each degrading subunit is illustrated separately and, in its entirety, and are fitted into one another to form the degrading subunit in Figure 5(b). In practice, the degrading subunit (11.ij) of Figure 5(b) can be produced by machining, etching, or 3D-printing a monolithic energy degrading unit (11.i), preferably with the support base (11b), or each degrading subunit (11.ij) individually and assembling them subsequently to form an energy degrading unit (and base support (11b)). Most preferably, the whole ridge filter is produced monolithically (i.e., requiring no assembly step).

## DEGRADING UNITS (11.i) MADE OF DIFFERENT MATERIALS

In a third embodiment illustrated in Figures 7(c) and 7(d), which can be combined with the previous embodiments of energy degrading units in the form of cavities or pins discussed supra, at least a first degrading subunit (11.11) of a first energy degrading unit (11.1) is made of a first material different from a second material of a second degrading subunit (11.ij) of the first or of a second energy degrading unit (11.1, 11.2). The first material having a value of the subunit water equivalent thickness per unit length (Wu) which is different from the second material, such as to vary, preferably decrease the value of the length (L11 = W11 / Wu) of the first degrading subunit (11.11), compared with the length of a corresponding first energy subunit (11.11) made of the second material.

Figure 7(c) shows an embodiment comprising first and second degrading subunits (11.i1, 11.i2) arranged concentrically. The second degrading subunit (11.i2) is enclosed within an annular first degrading subunit (11.i1). The first degrading subunit (11.i1) is made of a first material having a subunit water equivalent thickness per unit length (Wu) higher than the material forming the second degrading subunit (11.i2). It follows that, for a same length (Li1 = Li2) measured along the beam axis (Xi), the first degrading subunit (11.i1) absorbs more energy from the beam and results in a shift of the Bragg peak to a cell with a smaller cell water equivalent thickness (di1 < di2), lower than the cell water equivalent thickness (di2) crossing the second degrading subunit (11.i2).

Figure 7(d) shows a similar construction but with the first degrading subunit (11.i1) being made of a first material having a higher value of the subunit water equivalent thickness per unit length (Wu) than the second material the second degrading subunit (11.i2) is made of, which is positioned at the centre, surrounded by the second degrading subunit (11.i2). Consequently, the depth at which the Bragg peak occurs (di1 < di2) for the fraction of beam crossing the first degrading subunit (11.i1) situated at the centre of the energy degrading unit (11.i) is lower than the fraction crossing the second degrading subunit (11.i2) for a same length (Li1 = Li2) measured along the beam axis (Xi). The degrading subunits (11.i1, 11.i2) in Figures 7(c) and 7(d) are arranged concentrically. It is clear that other configurations are possible, such as for example arranging them side by side. Having a same length Li1 = Li2 is also an optimized situation. Since it is not possible to select materials spanning continuously a whole range of values of the subunit water equivalent thickness per unit length (Wu), it is difficult to get a selection of materials yielding the required cell water equivalent thicknesses (dij) and at the same time having the same length (Lij). Varying the materials, however, allows reducing the differences of heights between degrading subunits, yielding more compact and robust energy degrading units (11.i)

This embodiment, combining different materials having different subunit water equivalent thicknesses per unit length (Wu) can be applied to both cavity- and pin-shaped energy degrading units (11.i) to reduce the lengths (Lij) of the longest degrading subunits (11.ij) and increasing the lengths of the shortest degrading subunits (11.ij) to yield a shorter ridge filter (11) and to facilitate production and respect of tolerances, but selecting the dimensions most convenient for production.

For example, the choice of materials for each degrading subunit may be driven by the objective of yielding the length (L11) of the first degrading subunit (11.11) to be within $\pm 20\%$ of the length of the second degrading subunit (Lij), and so on. Preferably, the lengths (Lij) of all the degrading subunits (11.ij) of an energy degrading unit (11.i) have a same length (Lij) within a variation of $\pm 20\%$ of an average length (Lm,ij) (i.e., Lij = Lm,ij $\pm 20\%$ Vj). This way, a compact ridge filter can be obtained.

## RIDGE FILTER (11) AND PARTICLE ACCELERATOR COMPRISING THE RIDGE FILTER (11)

As illustrated in Figure 3(c), a ridge filter (11) is formed by a plurality of energy degrading units (11.i) arranged side by side on a base support (11b). The positions of the individual energy degrading units (11.i) correspond to the positions of the corresponding spots (Si), and their orientations is parallel to the corresponding beam axes (Xi).

All the beam axes (Xi) are represented parallel to each other in Figures 1(a), 1(b), 3(a), and 3(e). This is not exactly correct, since all the beam axes (Xi) initiate from a same point at the centre of the scanning magnets in the nozzle and are deviated to scan all the spots (Si) characterizing the treatment volume (V). This is represented in Figures 3(b), 3(d), and 3(f), wherein the angles of deviation are exaggerated forsake of clarity. The aperture angle of the cone enclosing all beam axes (Xi) depends on the distance between the scanning magnet and the treatment volume (V) and on the size of the treatment volume (V). Figure 3(b) shows a view of the irradiation system corresponding to the view of Figure 3(a), with the beam axes deviating from parallelism, taking account of the scanning effect of the beams. The aperture angle is exaggerated in Figure 3(b). Figure 3(c) shows a corresponding ridge filter (11) with pins jutting out of a support base (11b) and deviating from parallelism relative to one another. Figure 3(d) shows two energy degrading units formed by two corresponding orifices in the support base (11b). Here again, the aperture angle is exaggerated for sake of clarity.

In practice, the aperture angle is generally within $\pm 5°$, preferably within $\pm 3°$, more preferably within $\pm 1°$ from

the irradiation axis (X). For this reason, although not strictly correct, representing the beam axes (Xi) parallel to one another in the Figures is an acceptable simplification of reality.

Figures 3(e) and 3(f) show a similar cubic element of the treatment volume (V) comprising a number of subvolumes (Vi). Subvolumes (V1, V4, and V5) are visible in a side cut, showing the positions of corresponding cells (Cij, with i = 1, 4, and 5, and j = 1 to 4). In Figure 3(e), the subvolumes (Vi) extend (substantially) parallel to one another and to the irradiation axis (X), and in Figure 3(f), the subvolumes extend along diverging beam axes (Xi) (again, the aperture angle is exaggerated for sake of clarity).

The degrading subunit (11.i1) of longest length (Li1) measured along the corresponding beam axis (Xi) absorbs more energy of the beam (100.i) than the shorter degrading subunits. The longest degrading subunit (11.i1) therefore determines the shortest cell water equivalent thickness (di1) defining the position of the Bragg peak closest to the skin (3s) of the patient. As the lengths (Lij) of the degrading subunits (11.ij) decrease, the corresponding cell water equivalent thicknesses (dij) increase, until the shortest degrading subunit (11.iN) of shortest length (LiN) which determines the cell water equivalent thickness (diN) of the Bragg peak most remote from the skin (3s) of the patient. The superimposition of all the Bragg peaks forms the SOBP which must be according to the treatment plan (cf. Figures 4(a) to 4(e) and 5(a) to 5(e)). The length (Lij) of each degrading subunit (11.ij) can easily be determined as discussed supra, as Lij = Wij / Wu = (WO - dij) / Wu (cf. Figure 2(b)).

The area (Aij) of each degrading subunit (11.ij) must be dimensioned such as to bring the required number of charged particles to deposit the predefined doses (Dij) into the corresponding cells at the respective cell water equivalent thicknesses (dij). Equation (1) is used to determine the value of the area (Aij) of a degrading subunit (11.ij),

$$\frac{\omega_{ij}}{\Sigma_j \omega_{ij}} = \frac{\iint_{Aij} F(y,z).dy.dz}{\iint_{Abi} F(y,z).dy.dz}. \tag{1}$$

In Equation (1), the normalized beam weight ($\omega_{ij}$) is equated to the normalized value of the integral of the beam fluence (F(y, z)) over the area (Aij) to be dimensioned. As the area (Aij) defines the boundary over which the integral at the numerator of Equation (1) is calculated, the area (Aij) can be determined for each degrading subunit (11.ij). As discussed supra, a preferred arrangement of the individual degrading subunits (11.ij) is to assemble them coaxially to form a corresponding energy degrading unit (11.i) (cf. Figures 4(a) to (e) and 5(a) to (e)). In this arrangement in series, the area (Aij) of a first degrading subunit (11.ij) has an annular geometry forming an annular step around the degrading subunit (11.i(j+1)) inscribed coaxially within the first degrading subunit. In an arrangement in parallel as illustrated in Figures 4(f) to 4(j), 5(h) to 5(j)) and in Figures 7(e) and 7(g), the areas (Aij) of the degrading subunits (11.ij) are the areas at a base of each degrading subunit.

In case of a dense array of spots (Si), wherein the spots (Si) are separated from one another by a distance (ds) smaller than or equal to 1.8 times, preferably 1.5 times the standard deviation ($\sigma$) of the fluence (Fi(y,z)) of the beam (100.i), the fluence of the beam going through the base area (Abi) is approximated to being constant over all values of the planes (Y, Z)j defining the boundary inscribing the volume (V). This configuration substantially simplifies the resolution of the integral at the numerator of Equation (1).

If the array of spots (Si) is less dense, such that the spots are separated from one another by a distance (ds) larger than 1.2, preferably larger than 1.5 times the standard deviation ($\sigma$) of the fluence (Fi(y,z)) of the beam (100.i) (i.e., ds > 1.2 $\sigma$), the fluence (Fi(y,z)) of the beam (100.i) going through the base area (Abi) is approximated to a Gaussian,

$$F_i(y,z) = A_i.e^{-\left(\frac{(y-y_i)^2}{\sigma_y^2} + \frac{(z-z_i)^2}{\sigma_z^2}\right)},$$

where (yi,zi) is the coordinate in the (Y,Z) plane of the position of a maximum (Ai) of the fluence of the spot (Si) and wherein in the case of a circular spot, then $\sigma_y = \sigma_z = \sigma$. The resolution of the integral at the numerator of Equation (1) is not as easy as in the case of a dense array of spots (Si) (i.e., ds < 1.8 $\sigma$ or < 1.5 $\sigma$), but can still be solved, at least numerically. If the spots are circular and $\sigma_y = \sigma_z = \sigma$, the resolution of Equation (1) is simplified.

**CONCLUDING REMARKS**

The method proposed herein to design and dimension a ridge filter (11) for single layer PBS painting of a treatment volume (V) is simple, reliable, and reproducible. The design with energy degrading units (11.i) in the form of cavities is more robust to production tolerances than pins formed by concentric degrading subunits (11.ij), as the central degrading subunit (11.i1) is at the same time the longest and thinnest of the whole energy degrading unit (11.i), rendering the accurate production thereof more complex. Configurations other than concentric are possible, such as stacked, reducing somehow this issue. The use of materials having a higher subunit water equivalent thickness per unit length (Wu) for the degrading subunits (11.ij) of longest lengths (Lij) is also a solution to reduce the problem of accurate production of long thin pins.

Starting from a treatment plan, an array of the spots (Si) can be defined as described supra, and the treatment volume (V) can be divided into subvolumes (Vi) (one per spot) and the subvolumes (Vi) into N cells (Cij) accordingly. The doses (Dij) to be deposited into the cells (Cij) are determined based on the treatment plan.

The ridge filter is designed comprising the same number of energy degrading units (11.i) as there are spots (Si). Each energy degrading unit (11.i) is formed by N degrading subunits (11.ij) of lengths (Lij) and area (Aij).

The lengths (Lij) of each degrading subunit (11.ij) are calculated as the ratio of the desired subunit water equivalent thickness (Wij) to the subunit water equivalent thickness per unit length (Wu), (i.e., Lij = Wij / Wu). The subunit water equivalent thickness is defined as, Wij = WO - dij, wherein WO is the maximum beam range and dij is the desired position of the Bragg peak at a centre of the corresponding cell (Cij). The lengths (Lij) must take account of the thickness (Bi) of the support block (11b) supporting all the energy degrading units (11.i).

The area (Aij) of each degrading subunit (11.ij) is obtained by determining the area (Aij) over which the integral at the numerator of Equation (1) is computed. This operation can be carried out numerically.

The individual energy degrading units (11.i) are arranged on a support block (11b), such as to extend coaxially along the respective beam axes (Xi). The ridge filter can be produced by machining a block, by attaching individual pins to a support block (11b), by 3D-printing techniques and the like. The ridge filter (11) thus produced can be installed between the outlet of the accelerator of charged particles and the treatment volume (V) such that each subvolume (Vi) be coaxial with the corresponding beam axis (Xi). Irradiation by single layer PBS painting can start.

The ridge filter (11) designed by the method of the present invention is particularly suitable for treatment plans including FLASH-irradiation of at least a portion of the treatment volume (V) requiring doses (Dij) to be deposited into cells (Cij) at ultra-high deposition rates (HDR), by PBS, as it allows the whole treatment volume to be covered with a single paint layer, thus decreasing substantially the scanning time required to deposit doses (Dij) into each slice (Tj).

| REF | DESCRIPTION |
| --- | --- |
| 3s | Skin |
| 11 | Ridge filter |
| 11.i | Energy degrading unit |
| 11.ij | Degrading subunit |
| 100.i | Beam |
| Abi | Base area (Abi) of the degrading unit (11.i) |
| Aij | Area of base of degrading subunit 11.ij |
| Axij | Cross-sectional area of an energy degrading unit (11.i) at the level of a degrading subunit (11.ij) |
| Bi | Support block thickness along irradiation axis (X) |
| Cij | Cell |
| d0 | Shortest water equivalent thickness |
| d1 | Furthest water equivalent thickness |
| dij | Cell water equivalent thickness |
| Dij | Dose |
| F(y,z) | Fluence of the beam |
| Lij | Length along the beam axis (Xi) of a degrading subunit (11.ij) |
| Lsij | = Bs - Lij for energy degrading units in the form of cavities |
| Si | Spot |
| Tj | Slice |
| V | Treatment volume |
| Vi | Subvolume |

(continued)

| REF | DESCRIPTION |
|---|---|
| **W0** | Maximum beam range |
| **Wu** | Subunit water equivalent thickness per unit length |
| **X** | Irradiation axis |
| **Xi** | Beam axis |
| **X, Y, Z** | System of coordinates |
| **(Y,Z)** | Plane normal to the irradiation axis (X) |
| **(Y,Z)j** | Upstream plane of slice Tj |
| $\omega$**ij** | Weight of beam 100.i at slice Tj |

**Claims**

1. Method for designing a ridge filter of a charged particle accelerator, preferably an accelerator of protons, for depositing with beams of accelerated particles (100.i) specific doses (Dij) into specific locations within a treatment volume (V) of tissue comprising tumoral cells (3t) by pencil beam scanning (PBS), spot by spot (Si) according to a predefined treatment plan (TP), in a single painting layer defining the whole treatment volume (V), wherein the beams (100.i) extend along corresponding beam axes (Xi) substantially parallel to an irradiation axis (X), diverging from parallelism from the irradiation axis (X) by an angle comprised within ± 5°, preferably within ± 3°, and wherein the tissue is **characterized by** a maximum beam range (W0), defined as the water equivalent distance at which the beam stops propagating through the tissue, the method comprising the following steps,

   • defining a boundary inscribing the treatment volume (V) by defining areas (Aj) over upstream planes (Y,Z)j of N slices (Tj = T1 - TN) of thickness (dxj), wherein the planes (Y,Z)j are normal to the irradiation axis (X), wherein a shortest water equivalent thickness (d0) and longest water equivalent thickness (d1) to a skin of a patient are defined as the points of the boundary closest to and furthest away from the skin, respectively, measured along the irradiation axis (X),
   • defining an array of subvolumes (Vi), each subvolume extending parallel to the corresponding beam axes (Xi) from the skin of the patient to the corresponding furthest water equivalent thickness (d1), and whose projection onto a plane (Y,Z) normal to the irradiation axis (X) defines an array of spots (Si) covering a whole area of a projection of the volume (V) onto the plane (Y,Z),
   • for each slice (Tj) of the N slices (T1-TN), comprised within a subvolume (Vi), defining a cell (Cij) defined as a portion of the subvolume (Vi) comprised within the corresponding slice (Tj),
   • for each cell (Cij) of the given subvolume (Vi), determining a cell water equivalent thickness (dij) from the skin (3s) to a geometrical centre of the cells (Cij), and attributing a beam weight ($\omega$ij) required for depositing into the cell (Cij) the specific dose (Dij) according to the TP, wherein the beam weight ($\omega$ij) is proportional to the number of charged particles at the cell water equivalent thickness (dij),
   • designing the ridge filter (11) with a set of energy degrading units (11.i), wherein each energy degrading unit (11.ij) is configured for reducing an initial energy (E0) of a corresponding beam (100.i) of charged particles of beam diameter (D100.i), coaxial with the corresponding beam axes (Xi) and subvolume (Vi) to reduced energies (Eij), such that the specific doses (Dij) are deposited at the cell water equivalent thicknesses (dij) into the corresponding cells (Cij) comprised within the subvolume (Vi) according to the TP, the energy degrading unit (11.i) of a given subvolume (Vi) being designed as follows:

      ∘ for each cell (Cij) of the subvolume (Vi), dimensioning a degrading subunit (11.ij) having a generalized cylindrical geometry of base of area (Aij) normal to the corresponding beam axis (Xi) and of generatrixes of length (Lij) parallel to the corresponding beam axis (Xi), the degrading subunit being made of a material having a subunit water equivalent thickness per unit length (Wu) along the corresponding beam axis (Xi), wherein the length (Lij) is determined such that the degrading subunit (11.i) has a subunit water equivalent thickness (Wij = Wu x Lij) equal to a product of the subunit water equivalent thickness per unit length (Wu) and of the length (Lij), wherein a sum of the subunit water equivalent thickness (Wij) and of the cell water equivalent thickness (dij) is equal to the maximum beam range (W0) (i.e., W0 = Wij + dij), and

∘ the area (Aij) of a degrading subunit (11.ij) is determined by equating a normalized beam weight ($\omega_{ij} / \Sigma_j \omega_{ij}$) with a ratio of an integral of a fluence (F(y,z)) over the subunit base area (Aij) to the same integral over a base area (Abi) of the degrading unit (11.i) ,

$$\frac{\omega_{ij}}{\Sigma_j \omega_{ij}} = \frac{\iint_{Aij} F(y,z).\,dy.\,dz}{\iint_{Abi} F(y,z).\,dy.\,dz} \qquad (1)$$

wherein the fluence F(y,z) is a number of charges per unit area of the beam (100.i) at a position (y,z) of the beam, and wherein the base area (Abi) is equal to a sum of the subunit areas (Aij) (i.e., Abi = $\Sigma_j$ *Aij*),
∘ combining the N degrading subunits (11.ij) to obtain the energy degrading unit (11.i) designed for degrading the energy of the beam (100.i) such as to deposit the required doses (Dij) into the subvolume (Vi),

• Designing the energy degrading units (11.i) corresponding to all remaining subvolumes (Vi) as defined supra.

wherein the expression *"water equivalent thickness"* (= WET) is defined as a thickness of water causing a same energy degradation of a particle beam as a given thickness of one or more materials crossed by the particle beam.

2. Method according to claim 1, wherein the specific doses (Dij) are to be deposited according to the treatment plan at ultra-high dose deposition rate (HDR) into at least a selection of the specific locations within the volume (V) of tissue, wherein HDR is defined as a dose deposition rate, HDR $\geq$ 1 Gy / s.

3. Method according to claim 1 or 2, wherein the spots (Si) of the array of spots are separated from one another by a distance (ds) smaller than or equal to 1.8 times a standard deviation ($\sigma$) of the fluence (Fi(y,z)) of the beam (100.i) at one single spot (i.e., ds $\leq$ 1.8 $\sigma$), preferably smaller than or equal to 1.5 $\sigma$, and wherein the fluence (F(y,z)) of the beam (100.i) going through the base area (Abi) is approximated to being constant over all values of the planes (Y, Z)j defining the boundary inscribing the volume (V).

4. Method according to claim 1 or 2, wherein the spots (Si) of the array of spots are separated from one another by a distance (ds) larger than 1.2, preferably larger than 1.5 times a standard deviation ($\sigma$) of the fluence (Fi(y,z)) of the beam (100.i) at a single spot (100.i) (i.e., ds > 1.2 $\sigma$) and wherein the fluence (Fi(y,z)) of the beam (100.i) going through the base area (Abi) is approximated to being a Gaussian,

$$F_i(y,z) = A_i.e^{-\left(\frac{(y-y_i)^2}{\sigma_y^2} + \frac{(z-z_i)^2}{\sigma_z^2}\right)},$$

where (yi,zi) is the coordinate in the (Y,Z) plane of the position of a maximum (Ai) of the fluence of the spot (Si) and wherein in the case of a circular spot, then $\sigma_y = \sigma_z = \sigma$.

5. Method according to any one of the preceding claims 1 to 4, wherein

• the energy degrading units (11.i) are in the form of orifices arranged side-by-side according to the array of spots (Si) in a support base (11b) of thickness (Bi) measured along the beam axis (Xi), each orifice extending from an aperture opening at a surface of the support base (11b) and penetrating to a given depth measured along the corresponding beam axes (Xi), wherein
• each energy degrading unit (11.i),

∘ is formed by one or more degrading subunits (11.ij, 11.i3, 11.i2, 11.i1) in the form of orifices having a generalized cylindrical geometry of cross-sectional areas (Ai), and extending along the corresponding beam axis (Xi) from the aperture in the support block (11b) over lengths (Lsij), such that Lij = Bi - Lsij and, wherein
∘ the degrading subunits (11.ij, 11.i3, 11.i2, 11.i1) are arranged within the base area (Abi).

6. Method according to claim 5, wherein an energy degrading unit (11.i) comprises at least two subunits (11.ij) which are arranged within the base area (Abi) in one of the following configurations,

• in a construction in series, wherein,

◦ the degrading subunits are aligned along the corresponding beam axes (Xi), by order of decreasing lengths (Lsij), preferably coaxially and with the orifice having the longest length (Lsi3) being positioned at a central position, and wherein
◦ the subunit base area (Aij) of a given degrading subunit (11.ij) is equal to a difference of cross sectional areas (Axij - Axi(j + 1)) of the cross-sectional area (Axij) between the given degrading unit (11.ij) and the cross-sectional area (Axi(j+1)) of the degrading unit (Axi(j+1)) circumscribed within the given degrading unit,

• in a construction in parallel, wherein the degrading subunits are arranged side-by-side within the base area (Abi), either without spaces between two degrading subunits, or with a space between two adjacent degrading subunits,
• In a mixed construction both in parallel and in series, wherein three or more degrading subunits (11.ij) are arranged both in series and in parallel, wherein one or more structures formed by two or more degrading subunits aligned in series along the corresponding beam axis (Xi) and, optionally; one or more individual degrading subunits, are arranged side-by-side within the base area (Abi).

7.  Method according to any one of the preceding claims 1 to 4, wherein

• the energy degrading units (11.i) are in the form of pins arranged side-by-side according to the array of spots (Si) and supported on a support base (11b) of thickness (Bi) measured along the beam axis (Xi), each pin extending from the support base along the corresponding beam axes (Xi), wherein
• each energy degrading unit (11.i),

◦ is formed by one or more degrading subunits (11.ij, 11.i3, 11.i2, 11.i1) having a generalized cylindrical geometry of cross-sectional areas (Aij), and extending along the corresponding beam axis (Xi) from the support base over lengths (Lsij), such that Lij = Bi + Lsij and, wherein
◦ the degrading subunits (11.ij, 11.i3, 11.i2, 11.i1) are arranged within the base area (Abi).

8.  Method according to claim 7, wherein an energy degrading unit (11.i) comprises at least two subunits (11.ij) which are arranged within the base area (Abi) in one of the following configurations,

• in a construction in series, wherein,

◦ the degrading subunits are aligned along the corresponding beam axis (Xi), by order of decreasing lengths (Lsij), preferably coaxially and with the pin having the longest length (Lsi1) being positioned at a central position, and wherein
◦ the subunit base area (Aij) of a given degrading subunit (11.ij) is equal to a difference of cross sectional areas (Axij - Axi(j-1)) of the cross sectional area (Axij) between the given degrading unit (11.ij) and the cross sectional area (Axi(j-1)) of the degrading unit (Axi(j-1)) circumscribed within the given degrading unit,

• in a mixed construction both in parallel and in series, wherein three or more degrading subunits (11.ij) are arranged both in series and in parallel, wherein one or more structures formed by two or more degrading subunits aligned in series along the corresponding beam axis (Xi) and, optionally; one or more individual degrading subunits, are arranged side-by-side within the base area (Abi).

9.  Method according to any one of the preceding claims 5 to 8, wherein at least a first degrading subunit (11.11) of a first energy degrading unit (11.1) is made of a first material different from a second material of a second degrading subunit (11.ij) of the first or of a second energy degrading unit (11.1, 11.2), the first material having a value of the subunit water equivalent thickness per unit length (Wu) which is different from the second material, such as to vary, preferably decrease the value of the length (L11 = W11 / Wu) of the first degrading subunit (11.11), compared with the length of a corresponding first energy subunit (11.11) made of the second material.

10. Method according to claim 9, wherein the length (L11) of the first degrading subunit (11.11) is within ±20% of the length of the second degrading subunit (Lij), and preferably, the lengths (Lij) of all the degrading subunits (11.ij) of an energy degrading unit (11.i) have a same length (Lij) within a variation of ± 20% of an average length (Lm,ij) (i.e., Lij = Lm,ij ± 20% Vj).

**Patentansprüche**

1. Verfahren zum Auslegen eines Ridge-Filters eines Beschleunigers für geladene Teilchen, vorzugsweise eines Beschleunigers von Protonen, um mit Strahlen von beschleunigten Teilchen (100.i) spezifische Dosen (Dij) in spezifische Stellen innerhalb eines Behandlungsvolumens (V) von Gewebe, das Tumorzellen (3t) umfasst, durch Bleistiftstrahlabtastung (PBS) Fleck für Fleck (Si) gemäß einem im Voraus definierten Behandlungsplan (TP) in eine einzelne Anstrichschicht, die das gesamte Behandlungsvolumen (V) definiert, zu deponieren, wobei die Strahlen (100.i) sich entlang von korrespondierenden Strahlachsen (Xi) im Wesentlichen parallel zu einer Bestrahlungsachse (X), abweichend von der Parallelität von der Bestrahlungsachse (X) um einen Winkel, der innerhalb von $\pm 5°$, vorzugsweise innerhalb von $\pm 3°$ enthalten ist, erstrecken und wobei das Gewebe durch einen maximalen Strahlbereich (W0) gekennzeichnet ist, definiert als die Wasseräquivalentdistanz, bei der der Strahl aufhört, sich durch das Gewebe auszubreiten, das Verfahren die folgenden Schritte umfassend:

   • Definieren einer Begrenzung, die das Behandlungsvolumen (V) einbeschreibt, durch Definieren von Flächen (Aj) über vorgelagerten Ebenen (Y,Z)j von N Scheiben (Tj = T1 - TN) der Dicke (dxj), wobei die Ebenen (Y,Z)j normal zu der Bestrahlungsachse (X) sind, wobei eine kürzeste Wasseräquivalentdicke (d0) und längste Wasseräquivalentdicke (d1) zu einer Haut eines Patienten als die Punkte der Begrenzung definiert sind, die am nächsten zur Haut bzw. am weitesten von ihr entfernt sind, gemessen entlang der Bestrahlungsachse (X),
   • Definieren einer Anordnung von Teilvolumen (Vi), wobei jedes Teilvolumen sich parallel zu den korrespondierenden Strahlachsen (Xi) von der Haut des Patienten zu der korrespondierenden am weitesten entfernten Wasseräquivalentdicke (d1) erstreckt, und deren Projektion auf eine Ebene (Y,Z) normal zu der Bestrahlungsachse (X) eine Anordnung von Flecken (Si) definiert, die eine gesamte Fläche einer Projektion des Volumen (V) auf die Ebene (Y,Z) abdeckt,
   • für jede Scheibe (Tj) der N Scheiben (T1-TN), die innerhalb eines Teilvolumens (Vi) enthalten sind, Definieren einer Zelle (Cij), definiert als ein Abschnitt der Teilvolumen (Vi), die innerhalb der korrespondierenden Scheibe (Tj) enthalten sind,
   • für jede Zelle (Cij) der gegebenen Teilvolumen (Vi) Bestimmen einer Zellen-Wasseräquivalentdicke (dij) von der Haut (3s) zu einer geometrischen Mitte der Zellen (Cij) und Zuschreiben eines Strahlgewichts (wij), das zum Deponieren der spezifischen Dosis (Dij) gemäß dem TP in die Zelle (Cij) erforderlich ist, wobei das Strahlgewicht (wij) proportional zu der Anzahl von geladenen Teilchen bei der Zellen-Wasseräquivalentdicke (dij) ist,
   • Auslegen des Ridge-Filters (11) mit einem Satz von Energie degradierende Einheiten (11.i), wobei jede Energie degradierende Einheit (11.i) konfiguriert ist zum Reduzieren einer anfänglichen Energie (E0) eines korrespondierenden Strahls (100.i) von geladenen Teilchens des Strahldurchmessers (D100.i) koaxial mit den korrespondierenden Strahlachsen (Xi) und Teilvolumen (Vi) zu reduzierten Energien (Eij) derart, dass die spezifischen Dosen (Dij) an den Zellen-Wasseräquivalentdicken (dij) in die korrespondierenden Zellen (Cij), die innerhalb des Teilvolumens (Vi) gemäß der TP enthalten sind, deponiert werden, wobei die Energie degradierende Einheit (11.1) eines gegebenen Teilvolumens (Vi) wie folgt ausgelegt ist:

      ○ für jede Zelle (Cij) der Teilvolumen (Vi) Dimensionieren einer degradierenden Untereinheit (11.ij), die eine verallgemeinerte zylindrische Geometrie der Basis der Fläche (Aij) normal zu der korrespondierenden Strahlachse (Xi) und von Erzeugenden der Länge (Lij) parallel zu der korrespondierenden Strahlachse (Xi) aufweist, wobei die degradierende Untereinheit aus einem Material hergestellt ist, das eine Untereinheit-Wasseräquivalentdicke pro Einheitslänge (Wu) entlang der korrespondierenden Strahlachse (Xi) aufweist, wobei die Länge (Lij) derart bestimmt ist, dass die degradierende Untereinheit (11.i) eine Untereinheit-Wasseräquivalentdicke (Wij = Wu × Lij) gleich einem Produkt der Untereinheit-Wasseräquivalentdicke pro Einheitslänge (Wu) und der Länge (Lij) aufweist, wobei eine Summe der Untereinheit-Wasseräquivalentdicke (Wij) und der Zellen-Wasseräquivalentdicke (dij) gleich dem maximalen Strahlbereich (W0) ist (d. h. W0 = Vij + dij), und
      ○ die Fläche (Aij) einer degradierenden Untereinheit (11.ij) durch Gleichsetzen eines normalisierten Strahlgewichts ($\omega ij / \Sigma_j \, \omega_{ij}$) mit einem Verhältnis eines Integrals einer Fluenz (F(y,z)) über der Untereinheit-Basisfläche (Aij) zu demselben Integral über eine Basisfläche (Abi) der degradierenden Einheit (11.1) bestimmt wird,

$$\frac{\omega_{ij}}{\Sigma_j \omega_{ij}} = \frac{\iint_{Aij} F(y,z).\,dy.\,dz}{\iint_{Abi} F(y,z).\,dy.\,dz} (1)$$

wobei die Fluenz F(y,z) eine Anzahl von Ladungen pro Einheitsfläche des Strahls (100.i) an einer Position (y,z) des Strahls ist und wobei die Basisfläche (Abi) gleich einer Summe der Untereinheitsflächen (Ai) ist (d. h. Abi = $\Sigma_j$ A$_{ij}$),

o Kombinieren der N degradierenden Untereinheiten (11.ij), um die Energie degradierende Einheit (11.i) zu erhalten, die zum Degradieren der Energie des Strahls (100.i) derart ausgelegt ist, um die erforderlichen Dosen (Dij) in die Teilvolumen (Vi) zu deponieren,

• Auslegen der Energie degradierenden Einheiten (11.i) korrespondierend mit allen verbleibenden Teilvolumen (Vi), wie vorstehend definiert,

wobei der Ausdruck *"Wasseräquivalentdicke"* (= WET) definiert ist als eine Dicke von Wasser, das eine gleiche Energiedegradation eines Teilchenstrahls wie eine gegebene Dicke eines oder mehrerer Materialien, die von dem Teilchenstrahl durchquert werden, verursacht.

2. Verfahren nach Anspruch 1, wobei die spezifischen Dosen (Dij) gemäß dem Behandlungsplan bei einer ultrahohen Dosendeponierungsrate (HDR) in mindestens eine Auswahl der spezifischen Stellen innerhalb des Volumens (V) von Gewebe deponiert werden sollen, wobei HDR als eine Dosendeponierungsrate, HDR $\geq$ 1 Gy/s, definiert ist.

3. Verfahren nach Anspruch 1 oder 2, wobei die Flecken (Si) der Anordnung von Flecken voneinander durch eine Distanz (ds) kleiner als oder gleich dem 1,8-Fachen einer Standardabweichung ($\sigma$) der Fluenz (Fi(y,z)) des Strahls (100.i) an einem einzelnen Fleck (d. h. ds $\leq$ 1,8 $\sigma$), vorzugsweise kleiner als oder gleich 1,5 $\sigma$, getrennt sind und wobei die Fluenz (F(y,z)) des Strahls (100.i), der durch die Basisfläche (Abi) geht, näherungsweise über alle Werte der Ebenen (Y,Z)j, die die Begrenzung definieren, die das Volumen (V) einbeschreibt, konstant ist.

4. Verfahren nach Anspruch 1 oder 2, wobei die Flecken (Si) der Anordnung von Flecken voneinander durch eine Distanz (ds) größer als das 1,2-Fache, vorzugsweise größer als das 1,5-Fache einer Standardabweichung ($\sigma$) der Fluenz (Fi(y,z)) des Strahls (100.i) an einem einzelnen Fleck (100.i) (d. h. ds > 1,2 $\sigma$) getrennt sind und wobei die Fluenz (Fi(y,z)) des Strahls (100.i), der durch die Basisfläche (Abi) geht, näherungsweise ein Gaußscher Wert ist,

$$F_i(y, z) = A_j . e^{\left(\frac{(y-y_i)^2}{\sigma_z^2} + \frac{(z-z_i)^2}{\sigma_y^2}\right)},$$

wobei (yi,zi) die Koordinate in der (Y,Z)-Ebene der Position eines Maximums (Ai) der Fluenz des Flecks (Si) ist und wobei im Fall eines kreisförmigen Flecks $\sigma_y = \sigma_z = \sigma$ ist.

5. Verfahren nach einem der vorhergehenden Ansprüche 1 bis 4, wobei

• die Energie degradierenden Einheiten (11.i) in der Form von Öffnungen sind, angeordnet Seite an Seite gemäß der Anordnung von Flecken (Si) in einer Stützbasis (11b) der Dicke (Bi), gemessen entlang der Strahlachse (Xi), jede Öffnung sich von einer Blendenöffnung an einer Oberfläche der Stützbasis (11b) erstreckt und bis zu einer gegebenen Tiefe eindringt, gemessen entlang den korrespondierenden Strahlachsen (Xi), wobei
• jede Energie degradierende Einheit (11.i)

o durch eine oder mehrere degradierende Untereinheiten (11.ij, 11.13, 11.i2, 11.11) in der Form von Öffnungen gebildet ist, die eine verallgemeinerte zylindrische Geometrie von Querschnittsflächen (Ai) aufweisen und sich entlang der korrespondierenden Strahlachse (Xi) von der Blende in dem Stützblock (11b) über Längen (Lsij) derart erstrecken, dass Lij = Bi - Lsij ist, und wobei
o die degradierenden Untereinheiten (11.ij, 11.13, 11.i2, 11.11) innerhalb der Basisfläche (Abi) angeordnet sind.

6. Verfahren nach Anspruch 5, wobei eine Energie degradierende Einheit (11.i) mindestens zwei Untereinheiten (11.ij) umfasst, die innerhalb der Basisfläche (Abi) in einer der folgenden Konfigurationen angeordnet sind:

• in einer Konstruktion in Reihe, wobei

o die degradierenden Untereinheiten entlang den korrespondierenden Strahlachsen (Xi) in der Reihenfolge abnehmender Längen (Lsij) ausgerichtet sind, vorzugsweise koaxial und wobei die Öffnung, die die größte Länge (Lsi3) aufweist, in einer mittleren Position positioniert ist, und wobei
o die Untereinheit-Basisfläche (Aij) einer gegebenen degradierenden Untereinheit (11.ij) gleich einer Differenz von Querschnittsflächen (Axij - Axi(j + 1)) der Querschnittsfläche (Axij) zwischen der gegebenen degradierenden Einheit (11.ij) und der Querschnittsfläche (Axi(j+1)) der degradierenden Einheit (Axi(j+1)), die innerhalb der gegebenen degradierenden Einheit einbeschrieben ist, ist,

• in einer parallelen Konstruktion, wobei die degradierenden Untereinheiten Seite an Seite innerhalb die Basisfläche (Abi) angeordnet sind, entweder ohne Zwischenräume zwischen zwei degradierenden Untereinheiten oder mit einem Zwischenraum zwischen zwei aneinander angrenzenden degradierenden Untereinheiten,
• in einer gemischten Konstruktion sowohl parallel als auch in Reihe, wobei drei oder mehr degradierende Untereinheiten (11.ij) sowohl in Reihe als auch parallel angeordnet sind, wobei eine oder mehrere Strukturen, gebildet durch zwei oder mehr degradierende Untereinheiten, in Reihe entlang der korrespondierenden Strahlachse (Xi) ausgerichtet sind, und wahlweise; eine oder mehrere einzelne degradierende Untereinheiten Seite an Seite innerhalb die Basisfläche (Abi) angeordnet sind.

7. Verfahren nach einem der vorhergehenden Ansprüche 1 bis 4, wobei

• die Energie degradierenden Einheiten (11.1) in der Form von Stiften Seite an Seite gemäß der Anordnung von Flecken (Si) angeordnet sind und auf einer Stützbasis (11b) der Dicke (Bi), gemessen entlang der Strahlachse (Xi), gestützt werden, wobei jeder Stift sich von der Stützbasis entlang den korrespondierenden Strahlachsen (Xi) erstreckt, wobei
• jede Energie degradierende Einheit (11.i)

o durch eine oder mehrere degradierende Untereinheiten (11.ij, 11.13, 11.i2, 11.11) gebildet ist, die eine verallgemeinerte zylindrische Geometrie von Querschnittsflächen (Aij) aufweisen und sich entlang der korrespondierenden Strahlachse (Xi) von der Stützbasis über Längen (Lsij) derart erstrecken, dass Lij = Bi + Lsij ist, und wobei
o die degradierenden Untereinheiten (11.ij, 11.13, 11.i2, 11.11) innerhalb der Basisfläche (Abi) angeordnet sind.

8. Verfahren nach Anspruch 7, wobei eine Energie degradierende Einheit (11.i) mindestens zwei Untereinheiten (11.ij) umfasst, die innerhalb der Basisfläche (Abi) in einer der folgenden Konfigurationen angeordnet sind:

• in einer Konstruktion in Reihe, wobei

o die degradierenden Untereinheiten entlang der korrespondierenden Strahlachse (Xi) in der Reihenfolge abnehmender Längen (Lsij) ausgerichtet sind, vorzugsweise koaxial und wobei der Stift, der die größte Länge (Lsi1) aufweist, in einer mittleren Position positioniert ist, und wobei
o die Untereinheit-Basisfläche (Aij) einer gegebenen degradierenden Untereinheit (11.ij) gleich einer Differenz von Querschnittsflächen (Axij - Axi(j-1)) der Querschnittsfläche (Axij) zwischen der gegebenen degradierenden Einheit (11.ij) und der Querschnittsfläche (Axi(j-1)) der degradierenden Einheit (Axi(j-1)), die innerhalb der gegebenen degradierenden Einheit einbeschrieben ist, ist,

• in einer gemischten Konstruktion sowohl parallel als auch in Reihe, wobei drei oder mehr degradierende Untereinheiten (11.ij) sowohl in Reihe als auch parallel angeordnet sind, wobei eine oder mehrere Strukturen durch zwei oder mehr degradierende Untereinheiten gebildet werden, ausgerichtet in Reihe entlang der korrespondierenden Strahlachse (Xi), und wahlweise; eine oder mehrere einzelne degradierende Untereinheiten Seite an Seite innerhalb der Basisfläche (Abi) angeordnet sind.

9. Verfahren nach einem der vorhergehenden Ansprüche 5 bis 8, wobei mindestens eine erste degradierende Untereinheit (11.11) einer ersten Energie degradierenden Einheit (11.1) aus einem ersten Material hergestellt ist, verschieden von einem zweiten Material einer zweiten degradierenden Untereinheit (11.ij) der ersten oder einer zweiten Energie degradierenden Einheit (11.1, 11.2), wobei das erste Material einen Wert der Untereinheit-Wasseräquivalentdicke pro Einheitslänge (Wu) aufweist, der von dem zweiten Material verschieden ist, um den Wert der Länge (L11 = W11 / Wu) der ersten degradierenden Untereinheit (11.11) im Vergleich mit der Länge einer korrespondierenden ersten Energie-Untereinheit (11.11), die aus dem zweiten Material hergestellt ist, zu variieren, vorzugsweise zu verringern.

**10.** Verfahren nach Anspruch 9, wobei die Länge (L11) der ersten degradierenden Untereinheit (11.11) innerhalb von 120% der Länge der zweiten degradierenden Untereinheit (Lij) ist und vorzugsweise die Längen (Lij) sämtlicher der degradierenden Untereinheiten (11.ij) einer Energie degradierenden Einheit (11.i) eine gleiche Länge (Lij) innerhalb einer Variation von ±20% einer durchschnittlichen Länge (Lm,ij) aufweisen (d. h. Lij = Lm,ij ± 20% ∀j).

## Revendications

**1.** Procédé pour concevoir un filtre en peigne d'un accélérateur de particules chargées, de préférence un accélérateur de protons, pour déposer avec des faisceaux de particules accélérées (100.i) des doses spécifiques (Dij) dans des emplacements spécifiques à l'intérieur du volume de traitement (V) de tissu comportant des cellules tumorales (3t) par balayage par faisceau filiforme ou « pencil beam scanning » (PBS), point par point (Si) selon un plan de traitement prédéfini (TP), dans une seule couche de peinture définissant le volume de traitement tout entier (V), les faisceaux (100.i) s'étendant le long d'axes de faisceaux correspondants (Xi) essentiellement parallèles à un axe d'irradiation (X), divergeant du parallélisme avec l'axe d'irradiation (X) d'un angle compris dans ± 5°, de préférence dans ± 3°, et le tissu étant **caractérisé par** une portée de faisceau maximum (W0), définie comme la distance équivalente en eau à laquelle le faisceau arrête de se propager à travers le tissu, ce procédé comprenant les étapes suivantes :

  • la définition d'une enveloppe circonscrivant le volume de traitement (V) en définissant des aires (Aj) sur des plans amont (Y, Z) de N tranches (Tj = T1 - TN) d'une épaisseur (dxj), les plans (Y, Z) étant normaux à l'axe d'irradiation (X), dans lequel une épaisseur équivalente en eau la plus courte (d0) et une épaisseur équivalente en eau la plus longue (d1) jusqu'à une peau d'un patient sont définies comme les endroits de l'enveloppe les plus proches et les plus éloignés de la peau, respectivement, mesurés le long de l'axe d'irradiation (X),
  • la définition d'une matrice de sous-volumes (Vi), chaque sous-volume s'étendant parallèlement aux axes des faisceaux correspondants (Xi) depuis la peau du patient jusqu'à l'épaisseur équivalente en eau la plus éloignée correspondante (d1), et dont la projection sur un plan (Y, Z) normal à l'axe d'irradiation (X) définit une matrice de points (Si) couvrant une surface entière d'une projection du volume (V) sur le plan (Y, Z),
  • pour chaque tranche (Tj) des N tranches (T1 - TN), comprise à l'intérieur d'un sous-volume (Vi), la définition d'une cellule (Cij) définie comme une partie du sous-volume (Vi) comprise à l'intérieur de la tranche correspondante (Tj),
  • pour chaque cellule (Cij) du sous-volume donné (Vi), la détermination d'une épaisseur équivalente en eau (dij) de cellule depuis la peau (3s) jusqu'à un centre géométrique des cellules (Cij), et l'attribution d'un poids de faisceau (ωij) requis pour déposer dans la cellule (Cij) la dose spécifique (Dij) selon le plan de traitement (TP), le poids du faisceau (ωij) étant proportionnel au nombre de particules chargées à l'épaisseur équivalente en eau (dij) de la cellule,
  • la conception du filtre en peigne (11) avec un ensemble d'unités de dégradation d'énergie (11.i), chaque unité de dégradation d'énergie (11.i) étant configurée de façon à réduire une énergie initiale (E0) d'un faisceau correspondant (100.i) de particules chargées d'un diamètre de faisceau (D100.i), coaxial avec les axes des faisceaux correspondants (Xi), et le sous-volume (Vi) en des énergies réduites (Eij), de manière à ce que les doses spécifiques (Dij) soient déposées aux épaisseurs équivalentes en eau (dij) des cellules dans les cellules correspondantes (Cij) comprises à l'intérieur du sous-volume (Vi) selon le TP, l'unité de dégradation d'énergie (11.i) d'un sous-volume donné (Vi) étant conçue de la manière suivante :

    o pour chaque cellule (Cij) du sous-volume (Vi), le dimensionnement d'une sous-unité de dégradation (11.ij) ayant une géométrie cylindrique généralisée de la base d'une surface (Aij) normale à l'axe de faisceau correspondant (Xi) et de génératrices d'une longueur (Lij) parallèles à l'axe du faisceau correspondant (Xi), cette sous-unité de dégradation étant faite en un matériau ayant une épaisseur équivalente en eau de sous-unité par unité de longueur (Wu) le long de l'axe du faisceau correspondant (Xi), la longueur (Lij) étant déterminée de manière à ce que la sous-unité de dégradation (11.i) ait une épaisseur équivalente en eau de sous-unité (Wij = Wu x Lij) égale à un produit de l'épaisseur équivalente en eau de sous-unité par unité de longueur (Wu) et de la longueur (Lij), une somme de l'épaisseur équivalente en eau de sous-unité (Wij) et de l'épaisseur équivalente en eau de cellule (dij) étant égale à la plage de faisceau maximum (W0) (c'est-à-dire que W0 = Wij + dij), et
    o la surface (Aij) d'une sous-unité de dégradation (11.ij) est déterminée en mettant en équation un poids de faisceau normalisé (ωij / Σj ωij) avec un rapport entre une intégrale d'une fluence (F(y, z)) sur la surface de la base de la sous-unité (Aij) et la même intégrale sur une surface de base (Abi) de l'unité de dégradation (11.i),

$$\frac{\omega_{ij}}{\sum_j \omega_{ij}} = \frac{\iint_{Aij} F(y,z).\,dy.\,dz}{\iint_{Abi} F(y,z).\,dy.\,dz} \qquad (1)$$

la fluence F(y, z) étant un nombre de charges par unité de surface du faisceau (100.i) dans une position (y, z) du faisceau, et la surface de la base (Abi) étant égale à une somme des surfaces des sous-unités (Aij) (c'est-à-dire que Abi = $\sum_j$ *Aij),*

o la combinaison des N sous-unités de dégradation (11.ij) afin d'obtenir l'unité de dégradation d'énergie (11.i) conçue pour dégrader l'énergie du faisceau (100.i) de manière à déposer les doses requises (Dij) dans le sous-volume (Vi),

• la conception des unités de dégradation d'énergie (11.i) correspondant à tous les sous-volumes (Vi) restants tels que définis ci-dessus,

l'expression « *épaisseur équivalente en eau »* ou « water equivalent thickness » en anglais (= WET) étant définie comme une épaisseur d'eau causant une même dégradation d'énergie d'un faisceau de particules qu'une épaisseur donnée d'un ou de plusieurs matériaux traversés par le faisceau de particules.

2.  Procédé selon la revendication 1, dans lequel les doses spécifiques (Dij) doivent être déposées selon le plan de traitement à une vitesse de dépôt de dose (HDR) ultra-élevée dans au moins une sélection des emplacements spécifiques à l'intérieur du volume (V) de tissu, la HDR étant définie comme une vitesse de dépôt de dose, HDR ≥ 1 Gy / s.

3.  Procédé selon la revendication 1 ou 2, dans lequel les points (Si) de la matrice de points sont séparés les uns des autres par une distance (ds) plus petite que ou égale à 1,8 fois un écart type ($\sigma$) de la fluence (Fi(y, z) du faisceau (100.i) en un seul point (c'est-à-dire que ds ≤ 1,8 $\sigma$), de préférence plus petite que ou égale à 1,5 $\sigma$, et dans lequel la fluence (Fi(y, z) du faisceau (100.i) traversant la surface de la base (Abi) est approximée comme étant constante sur toutes les valeurs des plans (Y, Z)j définissant l'enveloppe circonscrivant le volume (V).

4.  Procédé selon la revendication 1 ou 2, dans lequel les points (Si) de la matrice de points sont séparés les uns des autres par une distance (ds) plus grande que 1,2 fois, de préférence plus grande que 1,5 fois un écart type ($\sigma$) de la fluence (Fi(y, z) du faisceau (100.i) en un seul point (100.i) (c'est-à-dire que ds > 1,2 $\sigma$), et dans lequel la fluence (Fi(y, z) du faisceau (100.i) traversant la surface de la base (Abi) est approximée comme étant une Gaussienne,

$$F_i(y,z) = A_i.\,e^{-\left(\frac{(y-y_i)^2}{\sigma_y^2} + \frac{(z-z_i)^2}{\sigma_z^2}\right)},$$

(yi, zi) étant la coordonnée dans le plan (Y, Z) de la position d'un maximum (Ai) de la fluence du point (Si) et, dans le cas d'un point circulaire, alors $\sigma_y = \sigma_z = \sigma$.

5.  Procédé selon l'une quelconque des revendications précédentes 1 à 4, dans lequel

• les unités de dégradation d'énergie (11.i) sont sous la forme d'orifices disposés côte à côte selon la matrice de points (Si) dans une base de support (11b) d'une épaisseur (Bi) mesurée le long de l'axe du faisceau (Xi), chaque orifice s'étendant depuis une ouverture située à une surface de la base de support (11b) et pénétrant jusqu'à une profondeur donnée le long des axes des faisceaux correspondants (Xi), dans lequel
• chaque unité de dégradation d'énergie (11.i)

o est formée par une ou plusieurs sous-unités de dégradation (11.ij, 11.13, 11.12, 11.11) sous la forme d'orifices ayant une géométrie cylindrique généralisée de surfaces de sections transversales (Ai) et s'étendant le long d'un axe de faisceau correspondant (Xi) depuis l'ouverture dans le bloc de support (11b) sur les longueurs (Lsij), de telle sorte que Lij = Bi - Lsij, et dans lequel
o les sous-unités de dégradation (11.ij, 11.13, 11.12, 11.11) sont disposées à l'intérieur de la surface de la base (Abi).

6.  Procédé selon la revendication 5, dans lequel une unité de dégradation d'énergie (11.i) comporte au moins deux sous-unités (11.ij) qui sont disposées à l'intérieur de la surface de la base (Abi) dans une des configurations suivantes :

• dans une construction en série, dans laquelle

    ◦ les sous-unités de dégradation sont alignées le long des axes des faisceaux correspondants (Xi), par ordre de longueurs décroissantes (Lsij), de préférence coaxialement et avec l'orifice ayant la longueur la plus longue (Lsi3) étant positionné dans une position centrale, et dans laquelle
    o la surface de la base de sous-unité (Aij) d'une sous-unité de dégradation donnée (11.ij) est égale à une différence des surfaces de sections transversales (Axij - Axi(j + 1)) de la surface de section transversale (Axij) entre l'unité de dégradation donnée (11.ij) et la surface de section transversale (Axi(j + 1) de l'unité de dégradation d'énergie (11.i) circonscrite à l'intérieur de l'unité de dégradation donnée,

• dans une construction en parallèle, dans laquelle les sous-unités de dégradation sont disposées côte à côte à l'intérieur de la surface de la base (Abi), soit sans espaces entre deux sous-unités de dégradation, soit avec un espace entre deux sous-unités de dégradation adjacentes,
• dans une construction mixte à la fois en parallèle et en série, dans laquelle trois sous-unités de dégradation (11.ij) ou plus sont disposées à la fois en série et en parallèle, dans laquelle une ou plusieurs structures formées par deux sous-unités de dégradation ou plus sont alignées en série le long de l'axe du faisceau correspondant (Xi) et, facultativement, dans laquelle une ou plusieurs sous-unités de dégradation individuelles sont disposées côte à côte à l'intérieur de la surface de la base (Abi).

**7.** Procédé selon l'une quelconque des revendications précédentes 1 à 4, dans lequel

• les unités de dégradation d'énergie (11.i) sont sous la forme de tiges disposées côte à côte selon la matrice de points (Si) et supportées sur une base de support (11b) d'une épaisseur (Bi) mesurée le long de l'axe du faisceau (Xi), chaque tige s'étendant depuis la base de support le long des axes des faisceaux correspondants, dans lequel
• chaque unité de dégradation d'énergie (11.i)

    o est formée par une ou plusieurs sous-unités de dégradation (11.ij, 11.13, 11.12, 11.11) ayant une géométrique cylindrique généralisée de surfaces de sections transversales (Aij), et s'étendant le long de l'axe du faisceau correspondant (Xi) depuis la base de support sur des longueurs (Lsij), de telle sorte que Lij = Bi - Lsij, dans lequel
    o les sous-unités de dégradation (11.ij, 11.13, 11.12, 11.11) sont disposées à l'intérieur de la surface de la base (Abi).

**8.** Procédé selon la revendication 7, dans lequel une unité de dégradation d'énergie (11.i) comporte au moins deux sous-unités (11.ij) qui sont disposées à l'intérieur de la surface de la base (Abi) dans une des configurations suivantes :

• dans une construction en série, dans laquelle

    ◦ les sous-unités de dégradation sont alignées le long des axes des faisceaux correspondants (Xi), par ordre de longueurs décroissantes (Lsij), de préférence coaxialement et avec la tige ayant la longueur la plus longue (Lsi1) étant positionnée dans une position centrale, et dans laquelle
    o la surface de la base de sous-unité (Aij) d'une sous-unité de dégradation donnée (11.ij) est égale à une différence de surfaces de sections transversales (Axij - Axi(j - 1)) de la surface de section transversale (Axij) entre l'unité de dégradation donnée (11.ij) et la surface de section transversale (Axi(j - 1) de l'unité de dégradation (11.i) circonscrite à l'intérieur de l'unité de dégradation donnée,

• dans une construction mixte à la fois en parallèle et en série, dans laquelle trois sous-unités de dégradation (11.ij) ou plus sont disposées à la fois en série et en parallèle, dans laquelle une ou plusieurs structures sont formées par deux sous-unités de dégradation ou plus alignées en série le long de l'axe du faisceau correspondant (Xi) et, facultativement, dans laquelle une ou plusieurs sous-unités de dégradation individuelles sont disposées côte à côte à l'intérieur de la surface de la base (Abi).

**9.** Procédé selon l'une quelconque des revendications précédentes 5 à 8, dans lequel au moins une première sous-unité de dégradation (11.11) d'une première unité de dégradation d'énergie (11.i) est faite en un premier matériau différent d'un deuxième matériau d'une deuxième sous-unité de dégradation (11.ij) de la première ou d'une deuxième unité de dégradation d'énergie (11.1, 11.2), le premier matériau ayant une valeur de l'épaisseur équivalente en eau de sous-unité par unité de longueur (Wu) qui est différente du deuxième matériau, de manière à varier, de préférence diminuer, la valeur de la longueur (L11 = W11 / Wu) de la première sous-unité de dégradation (11.11), par rapport à la longueur d'une première sous-unité de dégradation d'énergie correspondante (11.11) faite dans le deuxième matériau.

10. Procédé selon la revendication 9, dans lequel la longueur (L11) de la première sous-unité de dégradation (11.11) correspond à $\pm$ 20 % près à la longueur de la deuxième sous-unité de dégradation (Lij), et, de préférence, les longueurs (Lij) de toutes les sous-unités de dégradation (11.ij) d'une unité de dégradation d'énergie (11.i) ont une même longueur (Lij) avec une variation de $\pm$ 20% d'une longueur moyenne (Lm,ij) (c'est-à-dire que Lij = Lm,ij $\pm$ 20% Vj).

FIG.1(a)

**EP 4 183 447 B1**

FIG.1(c)

$\sum D_{ij}$ (Gy)

$V_i$

FIG.1(b)

$X_k$ — 100.k
$X_i$ — 100.i — 11.i
$X_m$ — 100.m

11

3s

$S_{k0}$ $V_k$ $S_{k1}$ $V_i$ $S_{i1} = d_1$ $V_k$

T1 $T_j$ TN

$d_0 = S_{i0}$ $S_{m0}$ $\underline{V}$ $S_{m1}$

$d_0$ $T_j$

$S_{i0}$ $S_{i1}$ $d_1$

$dx_j$

Z
X

FIG.1(d)

$\sum D_{mj}$ (Gy)

$V_m$

$S_{m0}$ $S_{m1}$

$d_0$ $d_1$

FIG.2(a)

D (Gy)

100%

80%

E0

3s

0 $d_{ij}$ W0 $X_i$

FIG.2(b)

D (Gy)

$L_{ij}$

E0 $E_{ij}$ 3s

11.ij

0 $d_{ij}$ W0 $X_i$

$(W0 - d_{ij})$

$= L_{ij} \times W_u$

27

**FIG.3(a)**

11(i+2)
11(i-1)
3s
y
V(i+2)   X(i+2)
V(i+1)   X(i+1)
Vi       Xi
X(i-1)
11i      X(i-2)
11(i-1)
V(i-1)
11(i-2)   V(i-2)

**FIG.3(b)**

11(i+2)   11(i-1)
3s
y
V(i+2)   X(i+2)
V(i+1)   X(i+1)
Vi       Xi
X(i-1)
11i      X(i-2)
11(i-1)
11(i-2)  V(i-1)
V(i-2)

**FIG.3(c)**

11.(i+1)   11.(i+3)
11.i   11.(i+2)   11.(i+4)
11.i1
11.i2
11.i3
11b

**FIG.3(d)**

11(i+1)
X(i+1)   100.(i+1)
Xi       100.i
1
1
i

FIG.3(e)

FIG.3(f)

FIG.4(a)

$Ab1 = \sum_{j=1}^{3} A1j$

$Ab2 = \sum_{j=1}^{3} A2j$

$Abi = \sum_j Aij$

FIG.4(b)

FIG.4(c)
$Ai1 = Axi1 - Axi2$

FIG.4(d)
$Ai2 = Axi2 - Axi3$

FIG.4(e)
$Ai3 = Axi3$

30

**FIG.4(f)**

$$Ab1 = \sum_{j=1}^{3} A1j$$

$$Ab2 = \sum_{j=1}^{3} A2j$$

$$Abi = \sum_{j} Aij$$

**FIG.4(g)**

**FIG.4(h)**

$$Ai1 = Abi - Axi2 - Axi3$$

**FIG.4(i)**

$$Ai2 = Axi2$$

**FIG.4(j)**

$$Ai3 = Axi3$$

FIG.5(a)

$= \sum_{j=1}^{3} A1j$

$= \sum_{j=1}^{3} A2j$

$Abi = \sum_j Aij$

FIG.5(b)

$Ai3 = Axi3 - Axi2$

FIG.5(c)

$Ai2 = Axi2 - Axi1$

FIG.5(d)

$Ai1 = Axi1$

FIG.5(e)

FIG.5(f)

FIG.5(g)

FIG.5(h)

FIG.5(i)

FIG.5(j)

$Ab1 = \sum_{j=1}^{3} A1j$

$Ab2 = \sum_{j=1}^{3} A2j$

$Abi = \sum_j Aij$

$Ai3 = Aib - Axi1 - Axi2$

$Ai2 = Axi2$

$Ai1 = Axi1$

FIG.6(a)

FIG.6(b)

FIG.6(c)

FIG.6(d)

FIG.6(e)

EP 4 183 447 B1

FIG.7(a)

FIG.7(b)

FIG.7(c)

FIG.7(d)

FIG.7(e)

FIG.7(f)

FIG.7(g)

FIG.7(h)

FIG.7(i)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019136167 B

**Non-patent literature cited in the description**

- **SIMEONOV et al.** *Phys. Med. Biol.,* 2017, vol. 62, 7075
- **SAKAE et al.** *Med. Phys.,* 2000, vol. 27 (2), 368
- **SIMEONOV Y ; WEBER U ; PENCHEV P ; RINGBÆK TP ; SCHUY C ; BRONS S ; ENGENHART-CABILLIC R ; BLIEDTNER J ; ZINK K.** 3D range-modulator for scanned particle therapy: development, Monte Carlo simulations and experimental evaluation. *Phys Med Biol.,* 11 August 2017, vol. 62 (17), 7075-7096